# EUROPEAN PATENT APPLICATION

(11) **EP 4 279 076 A1**
(43) Date of publication of application: **22.11.2023**
(21) Application number: 22739527.4
(22) Date of filing: 17.01.2022
(51) Int. Cl.: A61K 31/7105

(54) **RNA MOLECULE, CHIMERIC NA MOLECULE, DOUBLE-STRANDED RNA MOLECULE, AND DOUBLE-STRANDED CHIMERIC NA MOLECULE**

(30) Priority: 15.01.2021 JP 2021005335
(71) Applicant: The University of Tokyo, Bunkyo-ku, Tokyo 113-8654 (JP)
(72) Inventor: UI-TEI, Kumiko, Tokyo 113-8654 (JP); KOBAYASHI, Yoshiaki, Tokyo 113-8654 (JP); SATO, Atsushi, Tokyo 113-8654 (JP); ASANO, Yoshimasa, Tokyo 113-8654 (JP); SUZUKI, Yuria, Tokyo 113-8654 (JP); LEDEY, Naomi, Tokyo 113-8654 (JP); SAIGO, Kaoru, Tokyo 168-0063 (JP); NATORI, Yukikazu, Yokohama-shi, Kanagawa 220-0012 (JP)
(74) Representative: Gill Jennings & Every LLP
(86) International application number: PCT/JP2022/001431
(87) International publication number: WO 2022/154122

(57) **Abstract**

The present invention is directed to provide novel RNA molecules. The RNA molecules are for RNA interference to target a mutant allele with a point mutation, in which (1) the molecule has a nucleotide sequence complementary to a nucleotide sequence of a coding region of the mutant allele; and (2) when counted from the base at the 5'-end in the nucleotide sequence complementary to the nucleotide sequence of the mutant allele, (2-1) a base at position 5 or 6 is mismatched with a base in the mutant allele; (2-2) a position 10 or 11 corresponds to the position of the point mutation; and (2-3) the group at the 2'-position of the pentose at positions 6-8 or positions 7 and 8 is modified with, e.g., OCH₃. In this RNA molecule, one or more ribonucleotides may be each replaced by, e.g., a deoxyribonucleotide. The molecule may form a double-stranded RNA with a complementary strand.

## Description

### Cross-reference to related applications

The present application claims priority to Japanese patent application No. 2021-005335, filed on January 15, 2021, which is hereby incorporated by reference.

### Technical field

The present invention relates to RNA molecules, chimeric NA molecules, double-stranded RNA molecules, and double-stranded chimeric NA molecules, for use in RNA interference.

### Related art

RNA interference is a simple and efficient way to specifically suppress the expression of a given target gene in cells.

siRNA, however, has been understood to suppress the expression of unintended targets (off-targeted genes) more than expected at the beginning (Jackson, A.L. et al., (2003) Nature Biotechnology vol. 21, pp. 635-637).

Particularly, it has been shown that the stronger the affinity of siRNA to the mRNA of the target, the greater the off-target effects are induced (Ui-Tei, K. et al. (2008) Nucleic Acids Res. vol. 36, pp. 7100-7109).

### Summary of the invention

### Problem to be solved by the invention

An object of the present invention is to provide novel RNA molecules, novel chimeric NA molecules, novel double-stranded RNA molecules, and novel double-stranded chimeric NA molecules.

### Means to solve the problem

During intensive efforts directed toward identifying RNA sequences with reduced off-target effects, the present inventors found that, when the molecules have a mismatch at position 5 or 6 and are modified at the 2'-position of the pentose in each of the ribonucleotides at positions 6-8 or positions 7 and 8, the contribution of nucleotides at positions 10 and 11 in RNA molecules to off-target effects becomes lower. Thus, in RNA interference targeted to a mutant allele of a gene which has a point mutation relative to its corresponding wild-type allele, the present inventors succeeded in mainly suppressing the expression of the mutant allele but not substantially suppressing the expression of the wild-type allele, by designing an RNA molecule in which the base at position 10 or 11 is at the position of the point mutation and is identical to the one in the mutant allele, and using a double-stranded RNA molecule with the aforementioned RNA molecule as a guide strand for RNA interference. The present invention was thus completed.

An aspect of the present invention is an RNA molecule for use in RNA interference to target a mutant allele of a gene, the mutant allele having a point mutation relative to a wild-type allele of the gene, wherein the gene is selected from the group consisting of *HTT*, *ATXN3*, *ZMYM3, CTNNB1, SMARCA4*, *SMO, AR*, *DNM2, KRT14*, *IL4R, MAPT*, *MS4A2*, *PABPN1, RHO, SCNIA, APOB, F12, CLCN7, SCN8A, PCSK9,* and *KRT6A*, and wherein the RNA molecule satisfies the followings:
(1) the molecule has a nucleotide sequence complementary to a nucleotide sequence of a coding region of the mutant allele except for a base specified in (2-1) below; and
(2) when counted from the base at the 5'-end of a nucleotide sequence complementary to the nucleotide sequence of the mutant allele,
   (2-1) a base at position 5 or 6 is mismatched with a base in the mutant allele;
   (2-2) a base at position 10 or 11 is at the position of the point mutation and is identical to the base at the position of the point mutation in the mutant allele; and
   (2-3) the group at the 2'-position of the pentose in each of ribonucleotides at positions 6-8 or positions 7 and 8 is independently modified with OCH₃, halogen, or LNA. The halogen may be fluorine. When the base at the 5'-end of the nucleotide sequence specified in (1) above is not adenine or uracil, the base may be replaced by adenine or uracil. When the base at the 3'-end of the nucleotide sequence specified in (1) above is not cytosine or guanine, the base may be replaced by cytosine or guanine. Any of the aforementioned RNA molecules may contain 13-28 nucleotides. Any of the aforementioned RNA molecules may be a chimeric NA molecule wherein one or more ribonucleotides are each replaced by a deoxyribonucleotide, an artificial nucleic acid, or a nucleic acid analog.

A further aspect of the present invention is a double-stranded RNA molecule including a guide strand and a passenger strand, wherein the guide strand is any one of the aforementioned RNA molecules, and the passenger strand is an RNA molecule with a sequence complementary to that of the RNA molecule of the guide strand. An overhang may be present at the 3'-end of the guide strand and/or at the 3'-end of the passenger strand. Either or both of the overhangs may be 1-3 nucleotide(s) long. Any of the aforementioned double-stranded RNA molecules may be a double-stranded chimeric NA molecule in which one or more ribonucleotides are each replaced by a deoxyribonucleotide, an artificial nucleic acid, or a nucleic acid analog.

A further aspect of the present invention is a method for producing an RNA molecule for use as a guide strand in RNA interference, including the step of producing any one of the aforementioned RNA molecules. The RNA molecule may be a chimeric NA molecule wherein one or more ribonucleotides are each replaced by a deoxyribonucleotide, an artificial nucleic acid, or a nucleic acid analog.

A further aspect of the present invention is a method for performing RNA interference to target a mutant allele of a gene in a cell containing a wild-type allele of the gene and the mutant allele of the gene, the mutant allele having a point mutation, wherein the method includes the step of introducing any one of the aforementioned RNA molecules, chimeric NA molecules, double-stranded RNA molecules, or any one of the aforementioned double-stranded chimeric NA molecules into the cell.

A further aspect of the present invention is a therapeutic agent for a patient with a disease or a prophylactic agent for a carrier of the disease, the patient or the carrier having wild-type and mutant alleles of a causative gene for the disease, the mutant allele having a point mutation and being responsible for the disease, wherein the therapeutic agent includes, as an active ingredient, any one of the aforementioned RNA molecules, chimeric NA molecules, double-stranded RNA molecules, or any one of the aforementioned double-stranded chimeric NA molecules. The disease may be one of those listed in Table 1 below.

**[Table 1]**

| Type | Gene | Gene name | Diseases |
|---|---|---|---|
| Triplet repeat diseases | *HTT* | Huntingtin | Huntington's disease |
| | *A TXN3* | ataxin 3 | Machado-Joseph disease (spinocerebellar ataxia-3) |
| Tumors | *ZMYM3* | zinc finger MYM-type containing 3 | medulloblastoma, lung adenocarcinoma , lung squamous cell carcinoma, small cell lung cancer, and medullary thyroid carcinoma |
| | *CTNNB1* | catenin beta 1 | non-small cell lung cancer (NSCLC), and hepatocellular carcinoma |
| | *SMARCA4* | SWI/SNF related, matrix associated, actin dependent regulator of chromatin, subfamily a, member 4 | ovarian clear cell carcinoma, SMARCA4-deficient thoracic sarcomatoid tumor, and lung tumor |
| | *SMO* | smoothened, frizzled class receptor | basal cell carcinoma, and medulloblastoma |
| | *AR* | androgen receptor | prostate cancer, and salivary duct carcinoma |
| Genetic diseases | *DNM2* | dynamin 2 | centronuclear myopathy-1 |
| | *KRT14* | keratin 14 | congenital epidermolysis bullosa |
| | *IL 4R* | interleukin 4 receptor | atopic susceptibility to disorders such as atopic dermatitis |
| | *MAPT* | microtubule associated protein tau | frontotemporal dementia (±parkinsonism symptoms), and Alzheimer's disease |
| | *MS4A2* | membrane spanning 4-domains A2 | atopic susceptibility to disorders such as atopic dermatitis, and childhood asthma |
| | *PABPN1* | poly(A) binding | oculopharyngeal muscular dystrophy |
| | | protein nuclear 1 | (OMPD) |
| | *RHO* | Rhodopsin | retinitis pigmentosa 4 |
| | *SCN1A* | sodium voltage-gated channel alpha subunit 1 | early infantile epileptic encephalopathy 6 (Dravet syndrome) |
| | *APOB* | apolipoprotein B | familial hypercholesterolemia, type 2 |
| | *F12* | coagulation factor XII | hereditary angioedema, type III |
| | *CLCN7* | chloride voltage-gated channel 7 | autosomal dominant osteopetrosis, type 2 |
| | *SCN8A* | sodium voltage-gated channel alpha subunit 8 | developmental and epileptic encephalopathy, 13, and Dravet syndrome |
| | *PCSK9* | proprotein convertase subtilisin/kexin type 9 | familial hypercholesterolemia, type 3 |
| | *KRT6A* | keratin 6A | pachyonychia congenita, and epidermolysis bullosa |

A further aspect of the present invention is a method for selecting an RNA molecule, a chimeric NA molecule, a double-stranded RNA molecule, or a double-stranded chimeric NA molecule for use in RNA interference to silence a target gene, the method including the steps of evaluating a gene-specific silencing ability of a plurality of the RNA molecules, the chimeric NA molecules, the double-stranded RNA molecules, or the double-stranded chimeric NA molecules, to the target gene by performing the aforementioned RNAi-based method *in vitro* using each of the plurality of any one of the aforementioned RNA molecules, chimeric NA molecules, double-stranded RNA molecules, or any one of the aforementioned double-stranded chimeric NA molecules; and selecting an RNA molecule, a chimeric NA molecule, a double-stranded RNA molecule, or a double-stranded chimeric NA molecule having at least a certain level of the gene-specific silencing ability.

### Brief description of the drawing

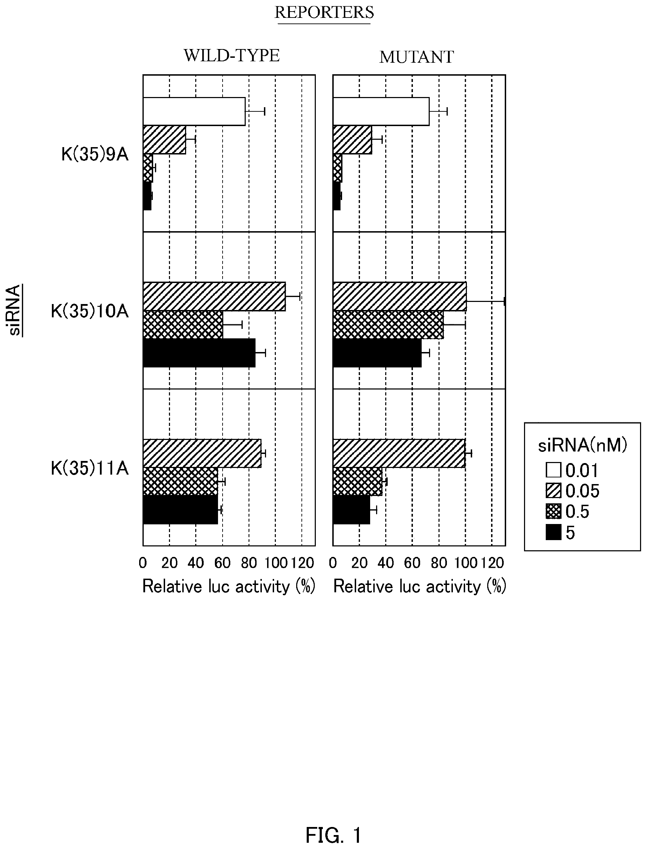

[Fig. 1] Fig. 1 shows graphs of the results on silencing abilities of siRNAs that target the K*-ras* gene, in each of which either one of positions 9-11 corresponded to the position of the point mutation, in an example of the present invention.

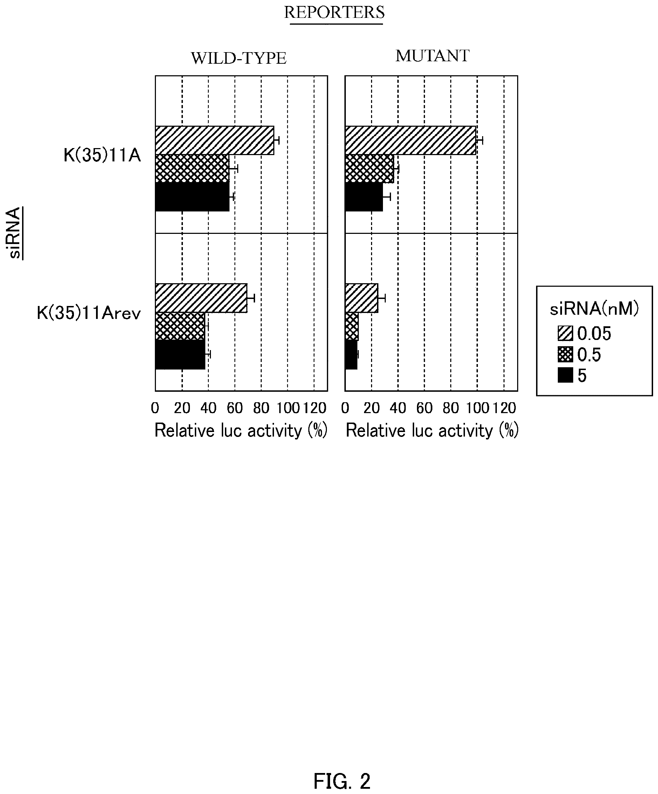

[Fig. 2] Fig. 2 shows graphs of the results on silencing abilities of an siRNA that targets the K*-ras* gene, in which position 11 corresponded to the position of the point mutation, the base at the 5'-end of the guide strand was changed from guanine to uracil, and the base at the 5'-end of the passenger strand was changed from uracil to guanine, in an example of the present invention.

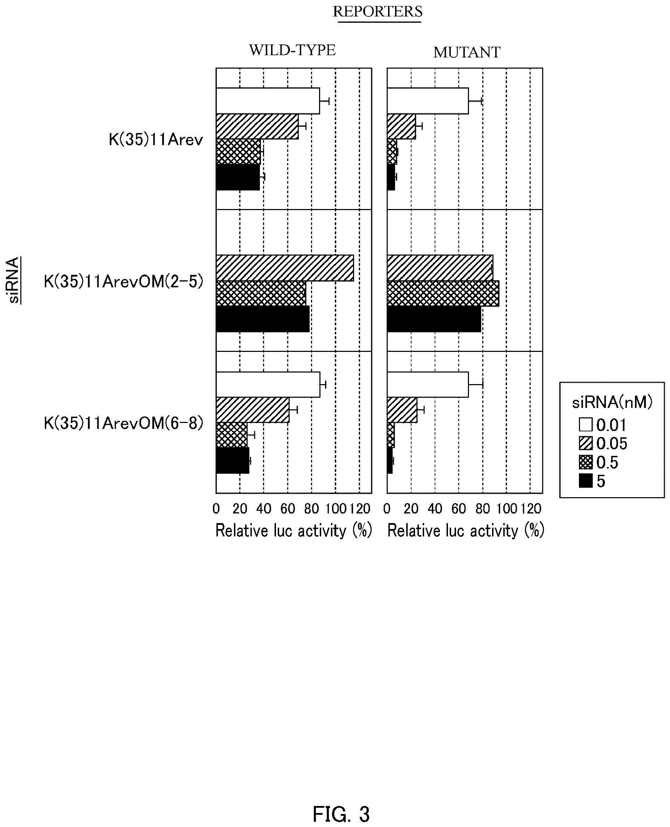

[Fig. 3] Fig. 3 shows graphs of the results on silencing abilities of siRNAs that target the K*-ras* gene, in each of which position 11 corresponded to the position of the point mutation, the base at the 5'-end of the guide strand was changed from guanine to uracil, the base at the 5'-end of the passenger strand was changed from uracil to guanine, and the group at the 2'-position of the pentose in each of ribonucleotides at positions 6-8 of the guide strand was replaced by OCH₃, in an example of the present invention.

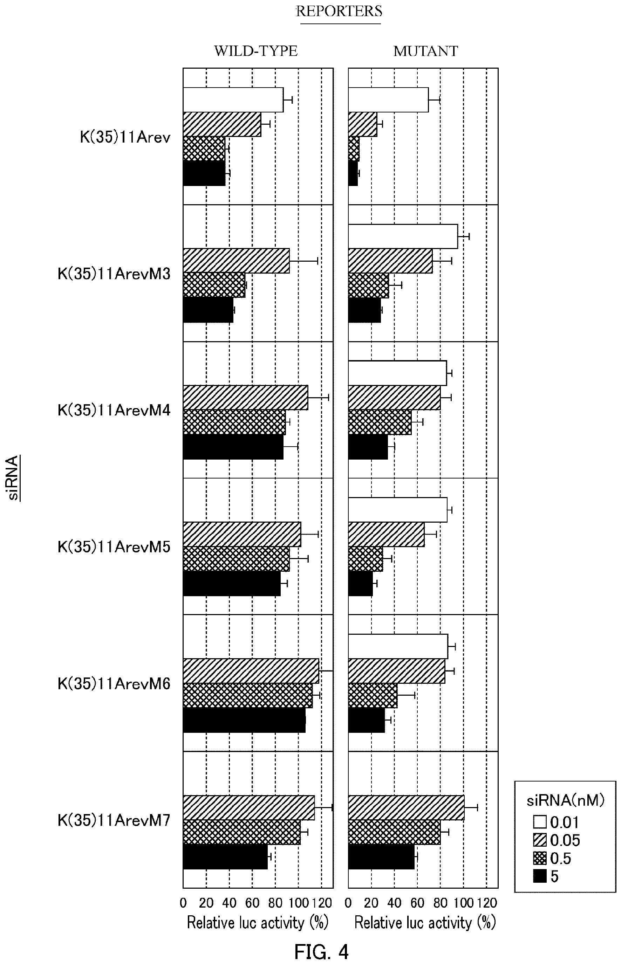

[Fig. 4] Fig. 4 shows graphs of the results on silencing abilities of siRNAs that target the K*-ras* gene, in each of which position 11 corresponded to the position of the point mutation, the base at the 5'-end of the guide strand was changed from guanine to uracil, the base at the 5'-end of the passenger strand was changed from uracil to guanine, and the base at either one of positions 3-7 of the guide strand was mismatched with that of the A-mutant allele, in an example of the present invention.

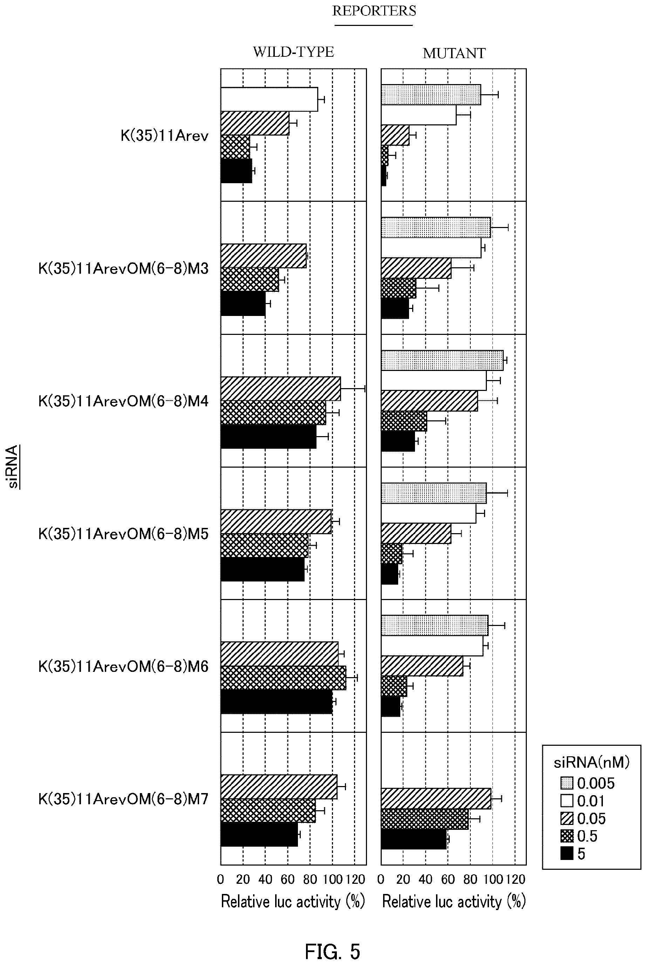

[Fig. 5] Fig. 5 shows graphs of the results on silencing abilities of siRNAs that target the K*-ras* gene, in each of which position 11 corresponded to the position of the point mutation, the base at the 5'-end of the guide strand was changed from guanine to uracil, the base at the 5'-end of the passenger strand was changed from uracil to guanine, the group at the 2'-position of the pentose in each of ribonucleotides at positions 6-8 of the guide strand was replaced by OCH₃, and the base at either one of positions 3-7 of the guide strand was mismatched with that of the A-mutant allele, in an example of the present invention.

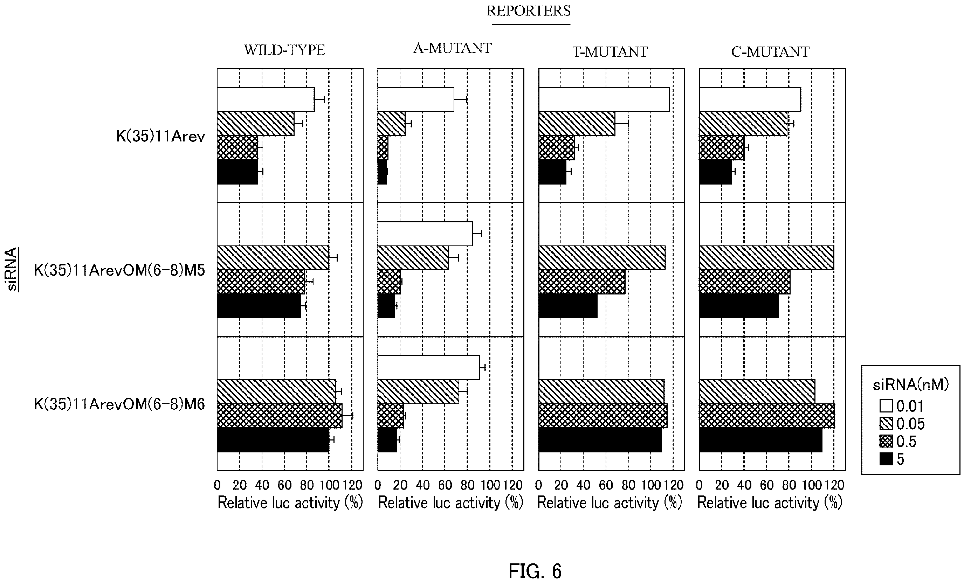

[Fig. 6] Fig. 6 shows graphs of the results on silencing abilities of siRNAs specific for the A-mutant allele of the K*-ras* gene, to the wild-type allele, the T-mutant allele of the K*-ras* gene (c. 35G>T), and the C-mutant allele of the K*-ras* gene (c. 35 G>C), in an example of the present invention.

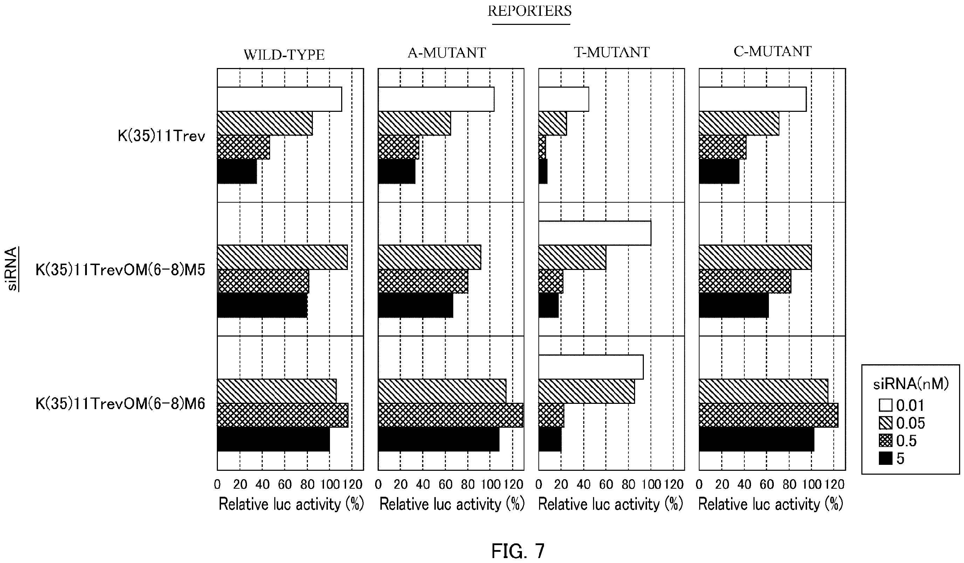

[Fig. 7] Fig. 7 shows graphs of the results on silencing abilities of siRNAs specific for the T-mutant allele of the K*-ras* gene, to the wild-type allele, and the A-and C-mutant alleles of the K*-ras* gene, in an example of the present invention.

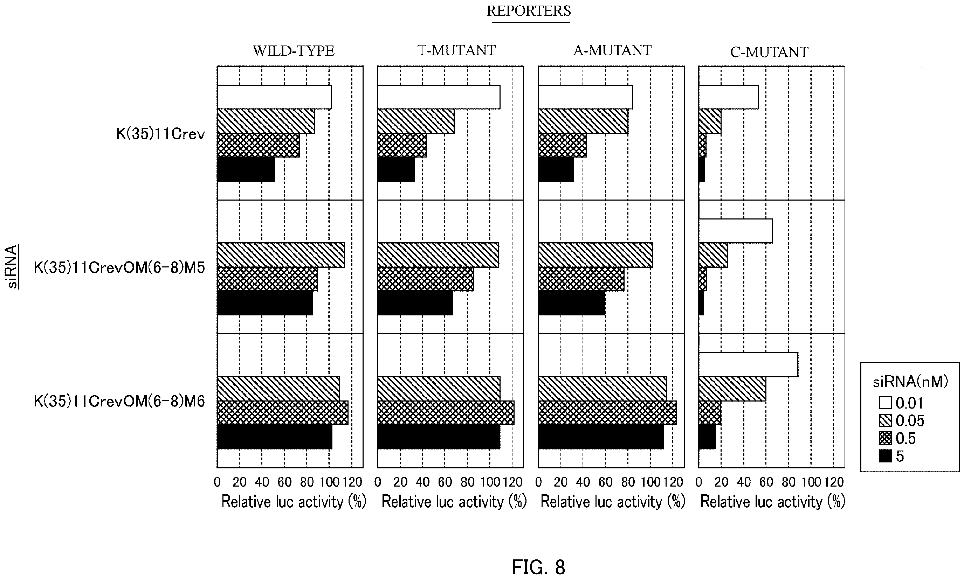

[Fig. 8] Fig. 8 shows graphs of the results on silencing abilities of siRNAs specific for the C-mutant allele of the K*-ras* gene, to the wild-type allele, and the A-and T-mutant alleles of the K*-ras* gene, in an example of the present invention.

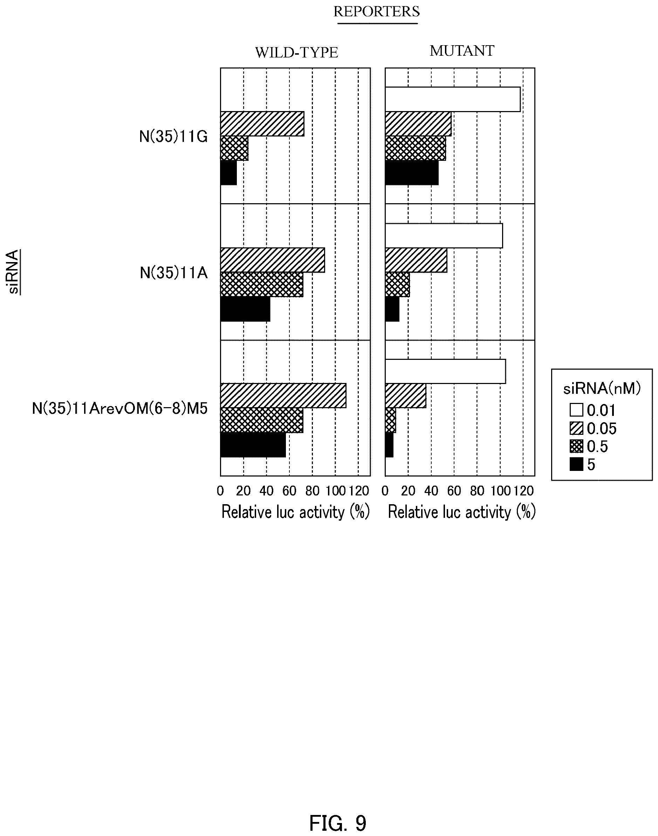

[Fig. 9] Fig. 9 shows graphs of the results on silencing abilities of siRNAs that target the point mutation in nt 35 of cDNA of the N*-ras* gene, in which position 11 corresponded to the position of the point mutation in the A-mutant allele of the N*-ras* gene (c. 35G>A) (hereinafter, referred to as the "A-mutant N35 allele"), the base at the 5'-end of the guide strand was changed from cytosine to uracil, the base at the 5'-end of the passenger strand was changed from adenine to guanine, the group at the 2'-position of the pentose in each of ribonucleotides at positions 6-8 of the guide strand was replaced by OCH₃, and the base at position 5 of the guide strand was mismatched with that of the A-mutant N35 allele, in an example of the present invention.

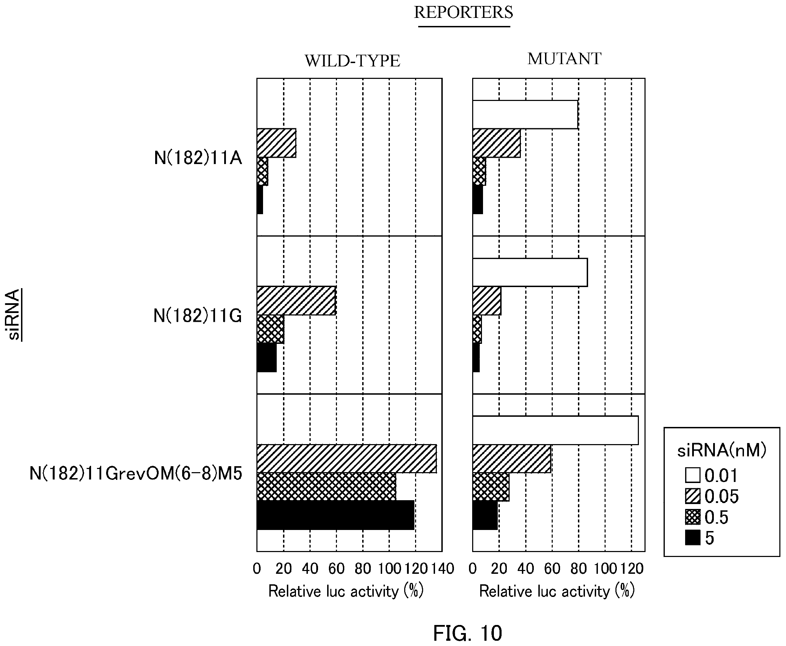

[Fig. 10] Fig. 10 shows graphs of the results on silencing abilities of siRNAs that target the point mutation in nt 182 of cDNA of the N*-ras* gene, in which position 11 corresponded to the position of the point mutation in the G-mutant allele of the N-*ras* gene (c. 182A>G) (hereinafter, referred to as the "G-mutant N182 allele"), the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 5 of the guide strand was mismatched with that of the G-mutant N182 allele, in an example of the present invention.

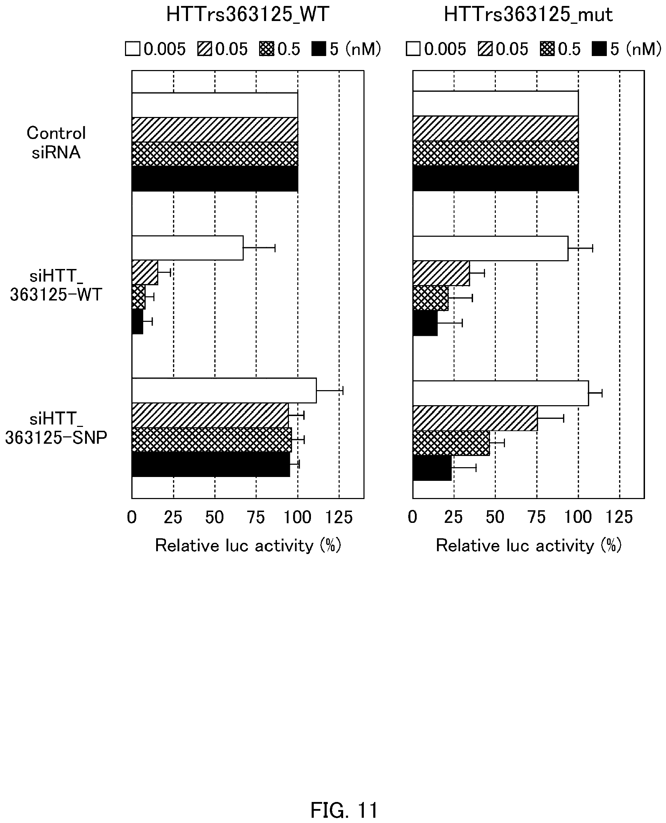

[Fig. 11] Fig. 11 shows graphs of the results on silencing abilities of a first siRNA that targets the *HTT* gene, in which position 11 corresponded to the position of the point mutation, the base at the 5'-end of the passenger strand was changed from cytosine to guanine, the group at the 2'-position of the pentose in each of ribonucleotides at positions 6-8 of the guide strand was replaced by OCH₃, and the base at position 6 of the guide strand was mismatched by changing it from uracil to adenine, in an example of the present invention.

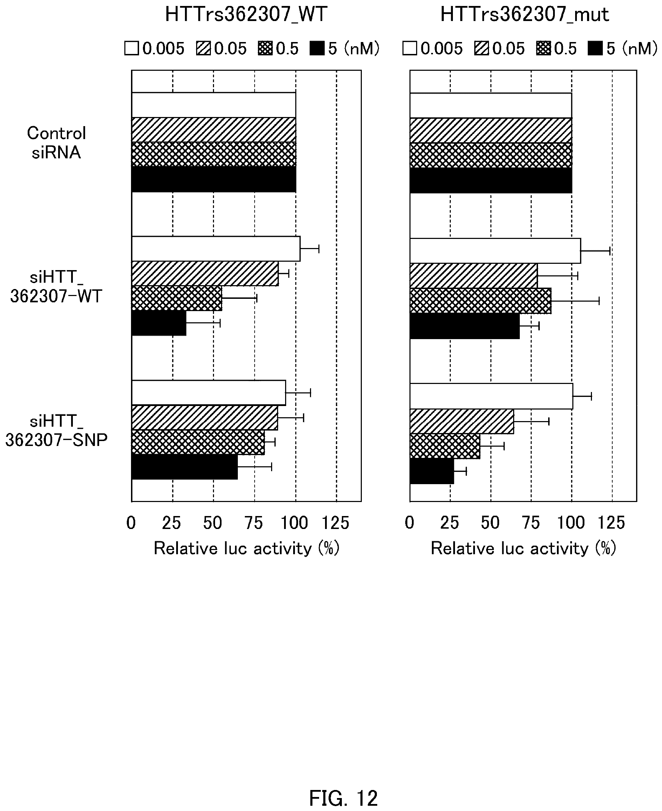

[Fig. 12] Fig. 12 shows graphs of the results on silencing abilities of a second siRNA that targets the *HTT* gene, in which position 11 corresponded to the position of the point mutation, the base at the 5'-end of the guide strand was changed from guanine to uracil, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

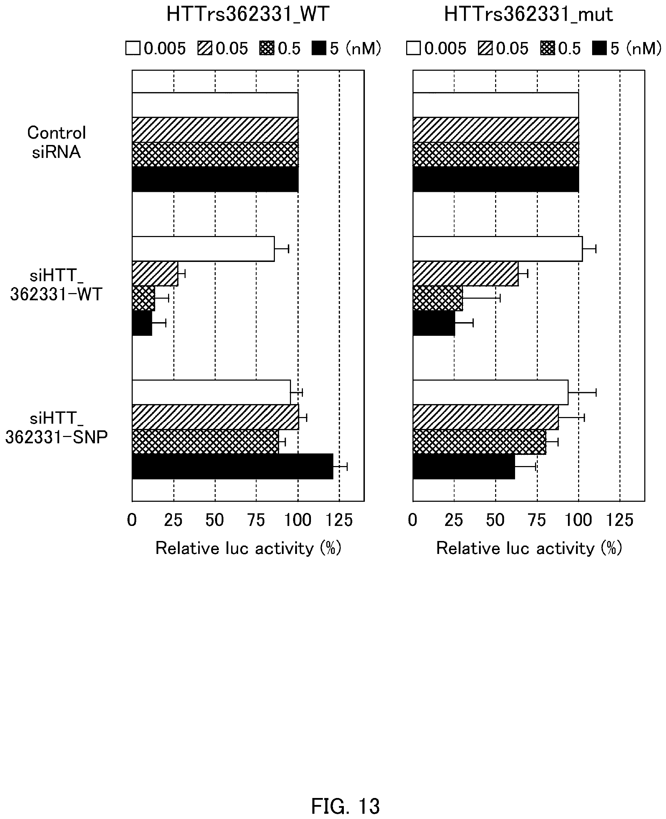

[Fig. 13] Fig. 13 shows graphs of the results on silencing abilities of a third siRNA that targets the *HTT* gene, in which position 11 corresponded to the position of the point mutation, the base at the 5'-end of the passenger strand was changed from cytosine to guanine, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

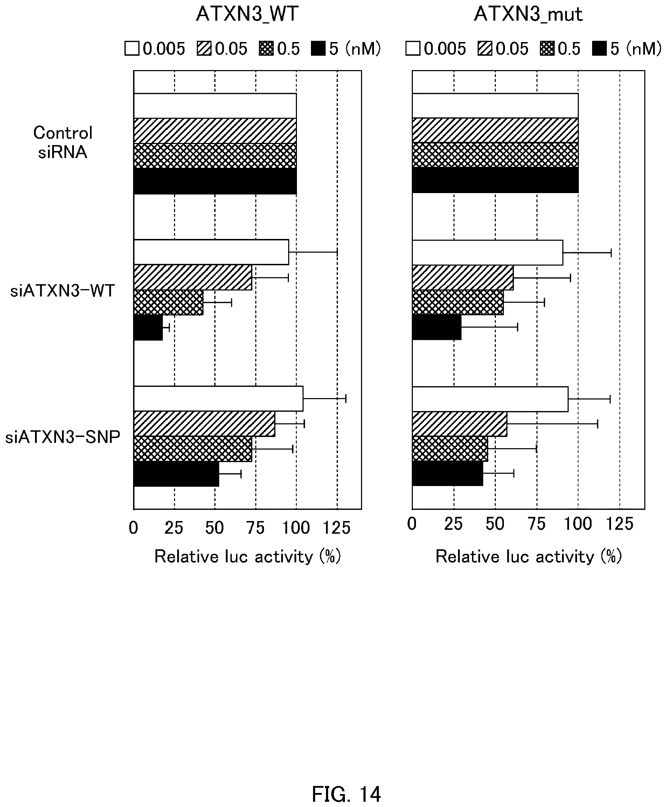

[Fig. 14] Fig. 14 shows graphs of the results on silencing abilities of siRNAs that target the *ATXN 3* gene, in each of which position 11 corresponded to the position of the point mutation, the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

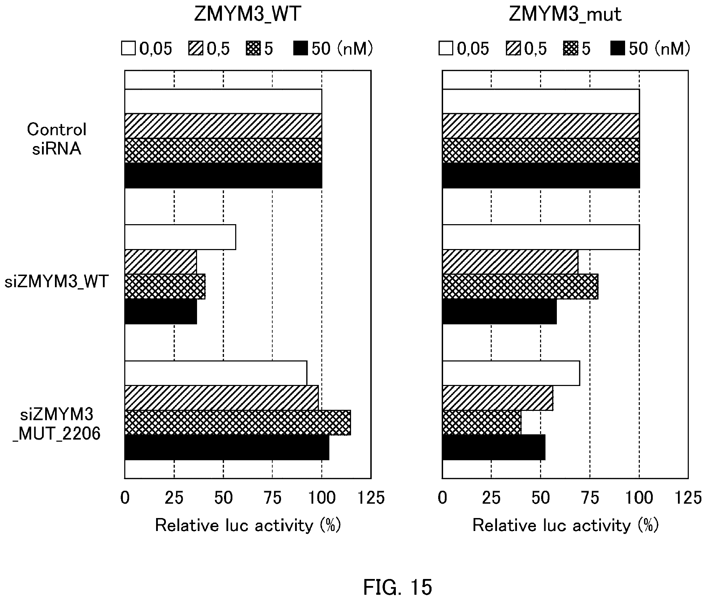

[Fig. 15] Fig. 15 shows graphs of the results on silencing abilities of siRNAs that target the *ZMYM3* gene, in each of which position 11 corresponded to the position of the point mutation, the base at the 5'-end of the guide strand was changed from adenine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

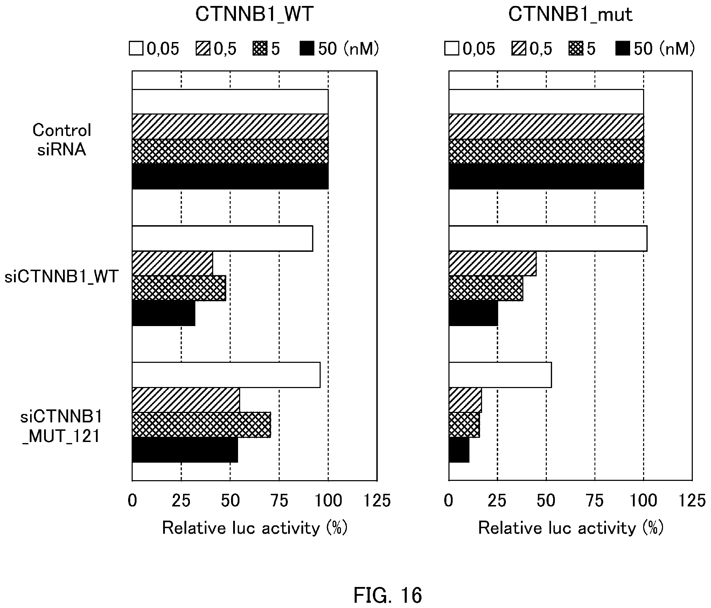

[Fig. 16] Fig. 16 shows graphs of the results on silencing abilities of siRNAs that target the *CTNNB1* gene, in each of which position 11 corresponded to the position of the point mutation, the base at the 5'-end of the guide strand was changed from guanine to uracil, the group at the 2'-position of the pentose in each of ribonucleotides at positions 6-8 of the guide strand was replaced by OCH₃, and the base at position 6 of the guide strand was mismatched by changing it from uracil to adenine, in an example of the present invention.

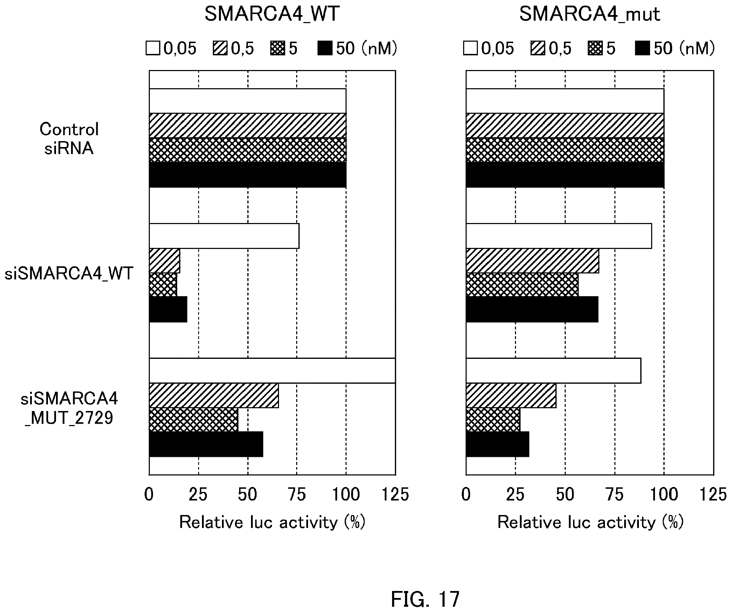

[Fig. 17] Fig. 17 shows graphs of the results on silencing abilities of siRNAs that target the *SMARCA4* gene, in each of which position 11 corresponded to the position of the point mutation, the base at the 5'-end of the guide strand was changed from cytosine to uracil, the group at the 2'-position of the pentose in each of ribonucleotides at positions 6-8 of the guide strand was replaced by OCH₃, and the base at position 6 of the guide strand was mismatched by changing it from uracil to adenine, in an example of the present invention.

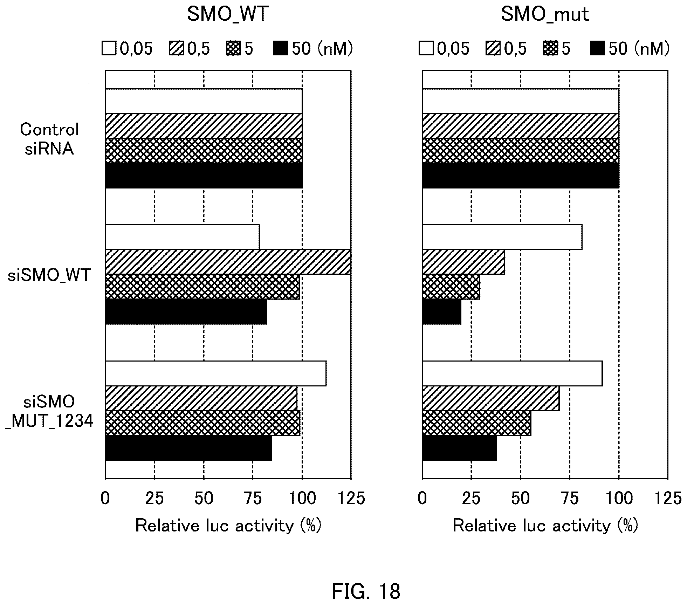

[Fig. 18] Fig. 18 shows graphs of the results on silencing abilities of siRNAs that target the *SMO* gene, in each of which position 11 corresponded to the position of the point mutation, the base at the 5'-end of the guide strand was changed from cytosine to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

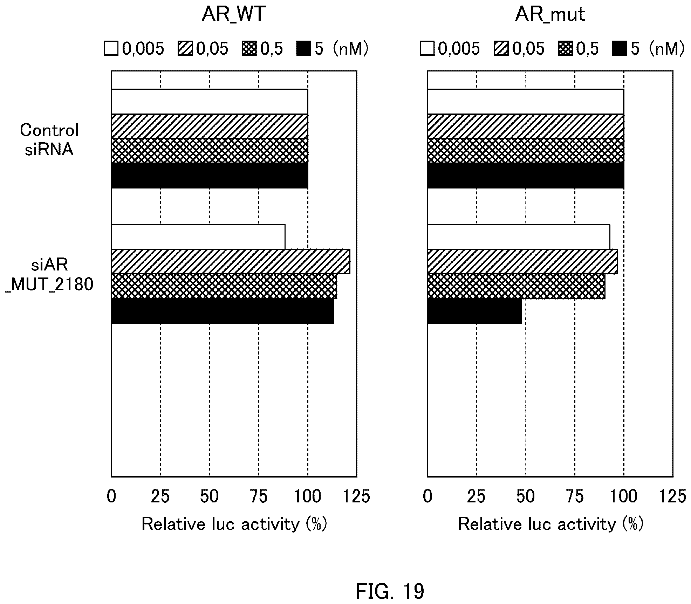

[Fig. 19] Fig. 19 shows graphs of the results on silencing abilities of siRNAs that target the *AR* gene, in each of which position 11 corresponded to the position of the point mutation, the base at the 5'-end of the guide strand was changed from guanine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

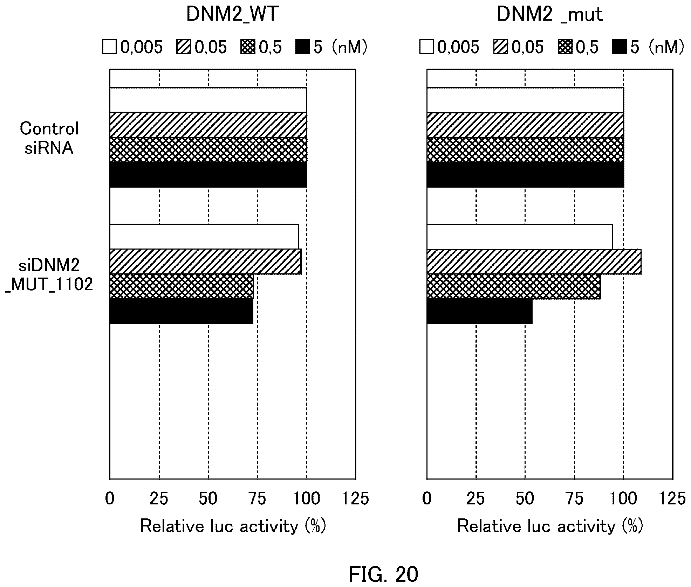

[Fig. 20] Fig. 20 shows graphs of the results on silencing abilities of siRNAs that target the *DNM2* gene, in each of which position 11 corresponded to the position of the point mutation, the base at the 5'-end of the guide strand was changed from guanine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

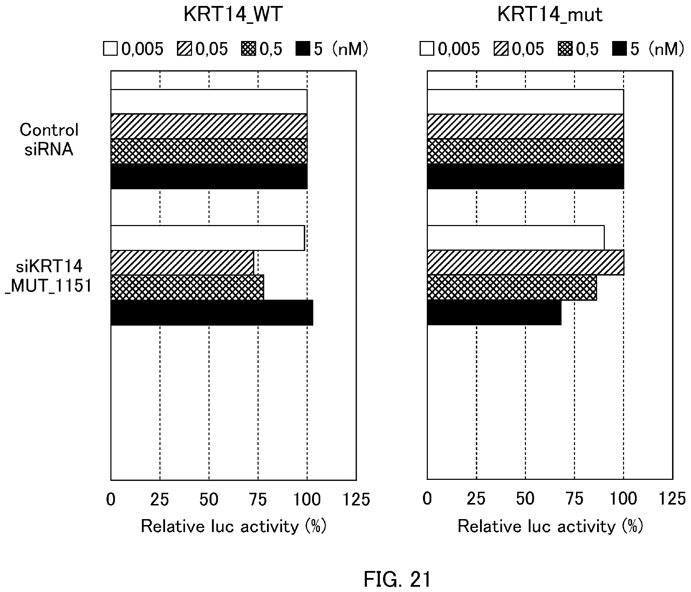

[Fig. 21] Fig. 21 shows graphs of the results on silencing abilities of siRNAs that target the *KRT14* gene, in each of which position 11 corresponded to the position of the point mutation, the base at the 5'-end of the guide strand was changed from guanine to uracil, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

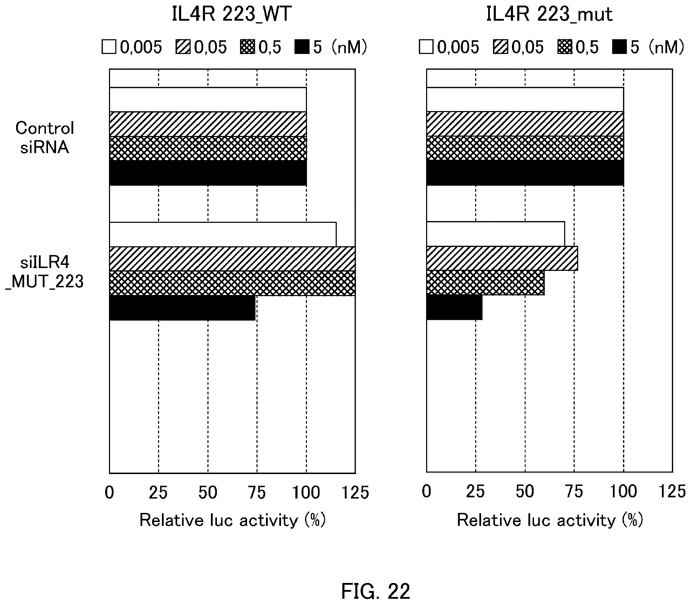

[Fig. 22] Fig. 22 shows graphs of the results on silencing abilities of a first siRNA that targets the *IL4R* gene, in which position 11 corresponded to the position of the point mutation, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

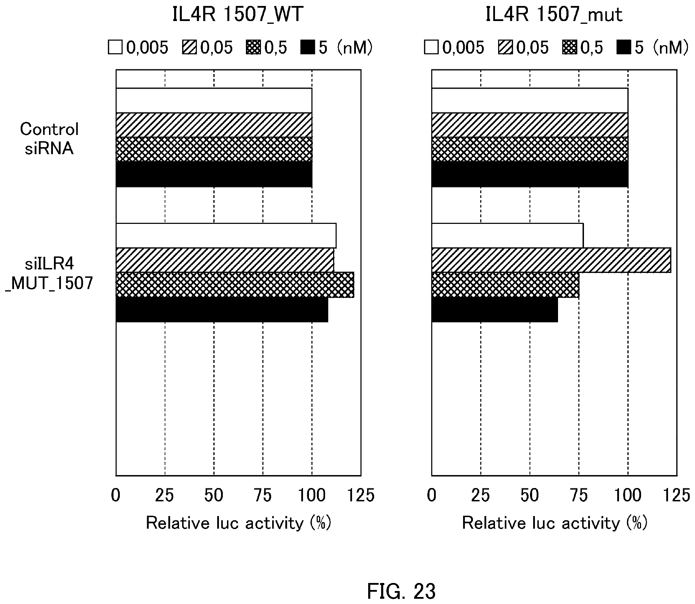

[Fig. 23] Fig. 23 shows graphs of the results on silencing abilities of a second siRNA that targets the *IL4R* gene, in which position 11 corresponded to the position of the point mutation, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

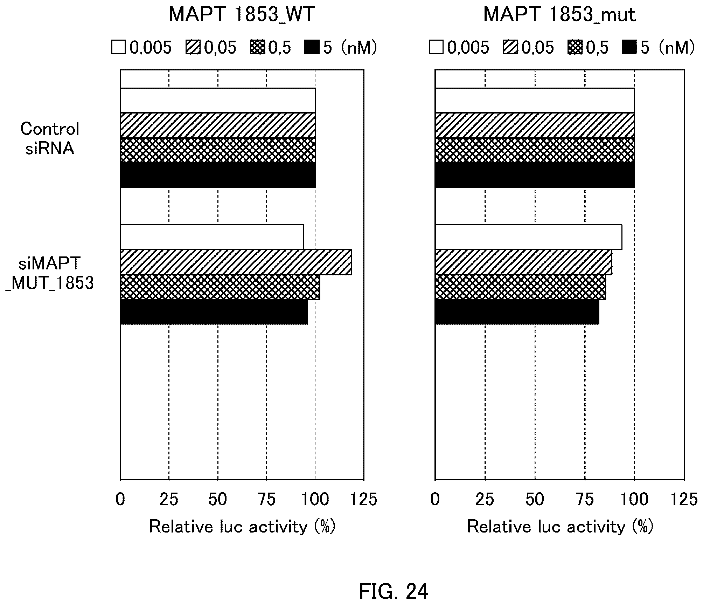

[Fig. 24] Fig. 24 shows graphs of the results on silencing abilities of siRNAs that target the *MAPT* gene, in each of which position 11 corresponded to the position of the point mutation, the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

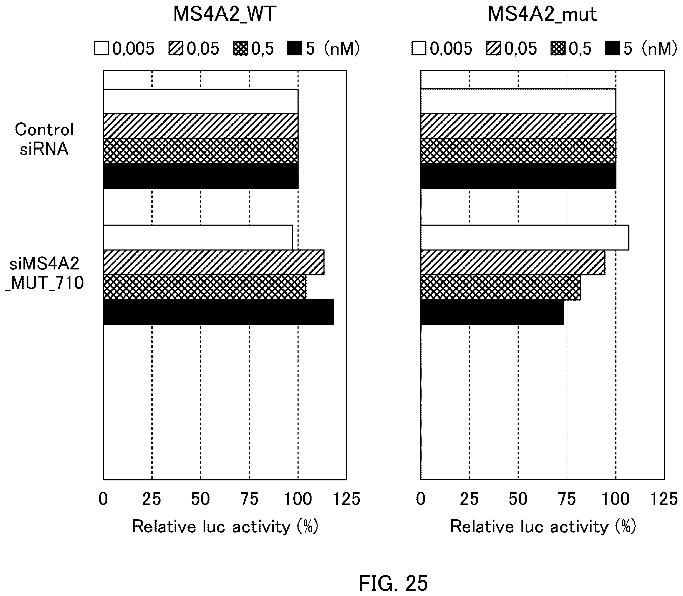

[Fig. 25] Fig. 25 shows graphs of the results on silencing abilities of siRNAs that target the *MS4A2* gene, in each of which position 11 corresponded to the position of the point mutation, the base at the 5'-end of the guide strand was changed from adenine to uracil, the base at the 5'-end of the passenger strand was changed from cytosine to guanine, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

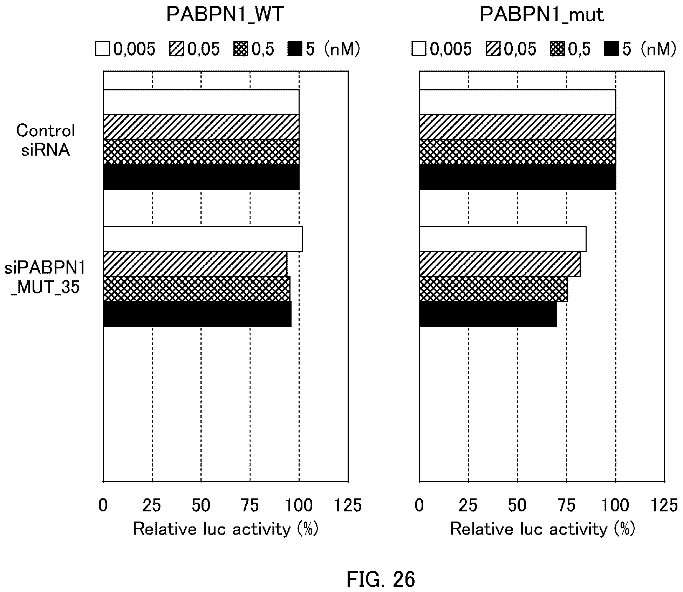

[Fig. 26] Fig. 26 shows graphs of the results on silencing abilities of siRNAs that target the *PABPN1* gene, in each of which position 11 corresponded to the position of the point mutation, the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

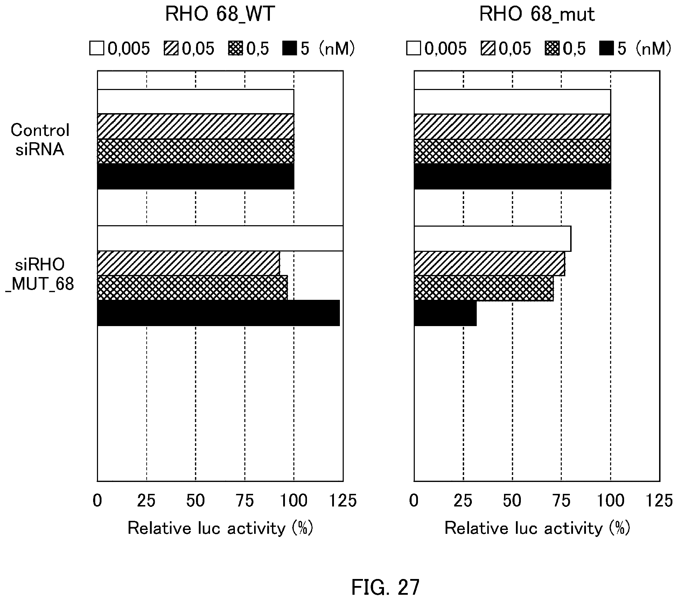

[Fig. 27] Fig. 27 shows graphs of the results on silencing abilities of a first siRNA that targets the *RHO* gene, in which position 11 corresponded to the position of the point mutation, the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

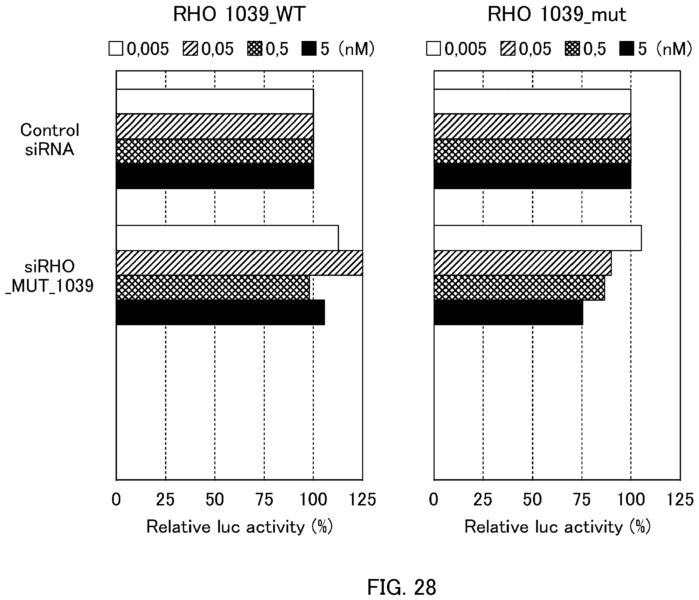

[Fig. 28] Fig. 28 shows graphs of the results on silencing abilities of a second siRNA that targets the *RHO* gene, in which position 11 corresponded to the position of the point mutation, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

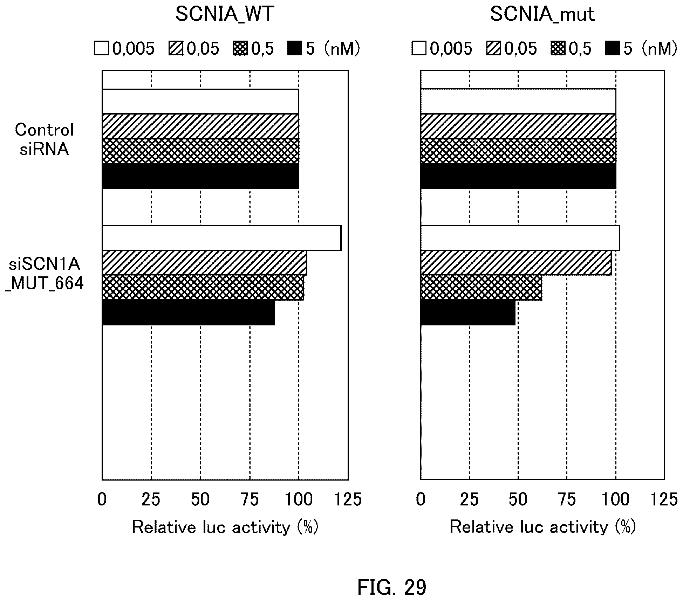

[Fig. 29] Fig. 29 shows graphs of the results on silencing abilities of siRNAs that target the *SCNIA* gene, in each of which position 11 corresponded to the position of the point mutation, the group at the 2'-position of the pentose in each of ribonucleotides at positions 6-8 of the guide strand was replaced by OCH₃, and the base at position 6 of the guide strand was mismatched by changing it from uracil to adenine, in an example of the present invention.

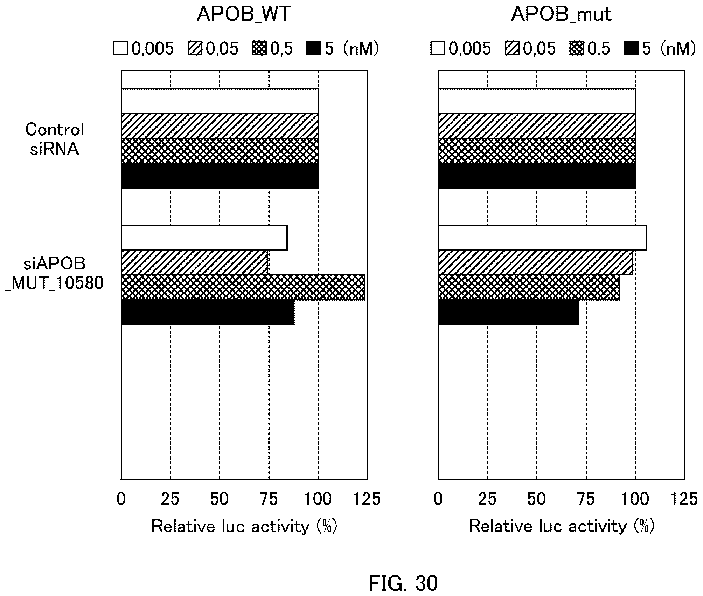

[Fig. 30] Fig. 30 shows graphs of the results on silencing abilities of siRNAs that target the *APOB* gene, in each of which position 11 corresponded to the position of the point mutation, the base at the 5'-end of the guide strand was changed from cytosine to uracil, the group at the 2'-position of the pentose in each of ribonucleotides at positions 6-8 of the guide strand was replaced by OCH₃, and the base at position 6 of the guide strand was mismatched by changing it from adenine to uracil, in an example of the present invention.

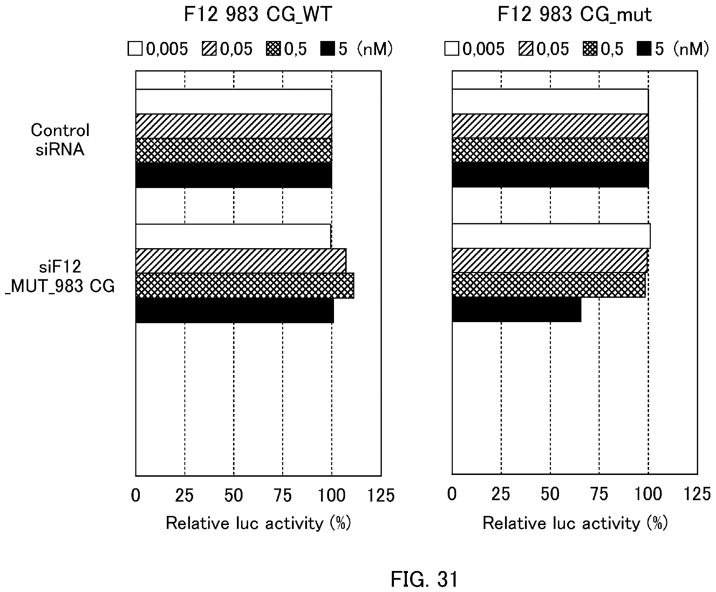

[Fig. 31] Fig. 31 shows graphs of the results on silencing abilities of siRNAs that target the *F12* gene, in each of which position 11 corresponded to the position of the point mutation, the base at the 5'-end of the guide strand was changed from guanine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

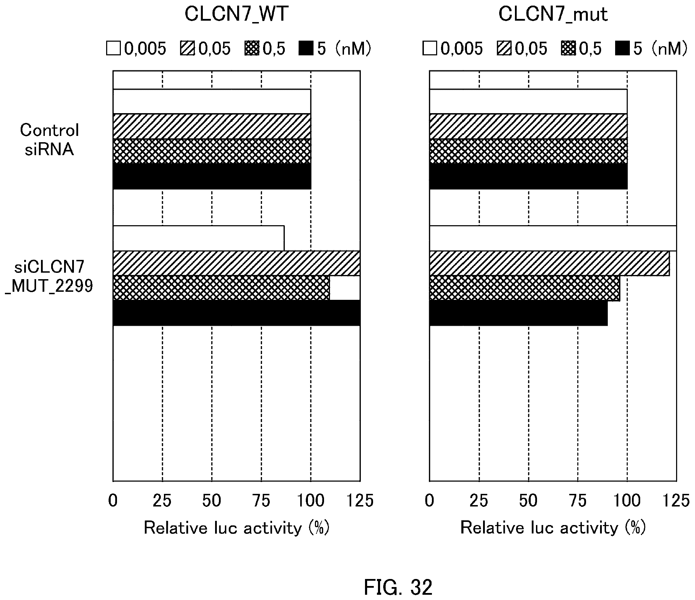

[Fig. 32] Fig. 32 shows graphs of the results on silencing abilities of siRNAs that target the *CLCN7* gene, in each of which position 11 corresponded to the position of the point mutation, the base at the 5'-end of the guide strand was changed from adenine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

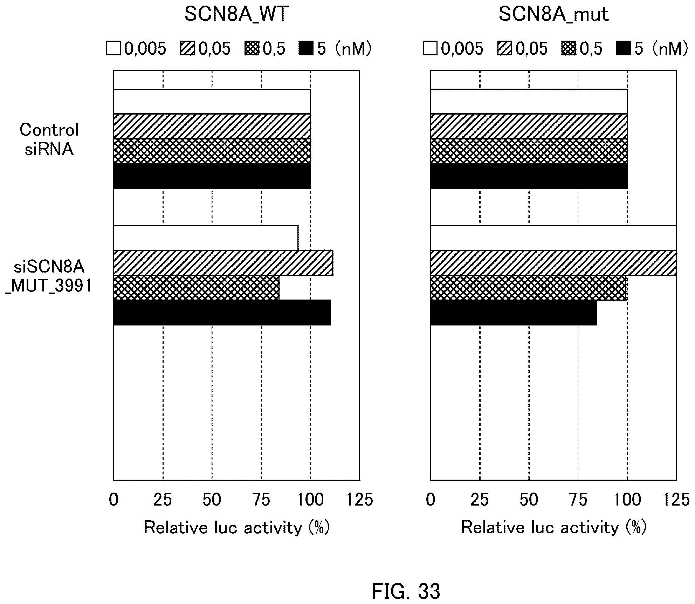

[Fig. 33] Fig. 33 shows graphs of the results on silencing abilities of siRNAs that target the *SCN8A* gene, in each of which position 11 corresponded to the position of the point mutation, the base at the 5'-end of the guide strand was changed from cytosine to uracil, the base at the 5'-end of the passenger strand was changed from uracil to guanine, the group at the 2'-position of the pentose in each of ribonucleotides at positions 6-8 of the guide strand was replaced by OCH₃, and the base at position 6 of the guide strand was mismatched by changing it from cytosine to guanine, in an example of the present invention.

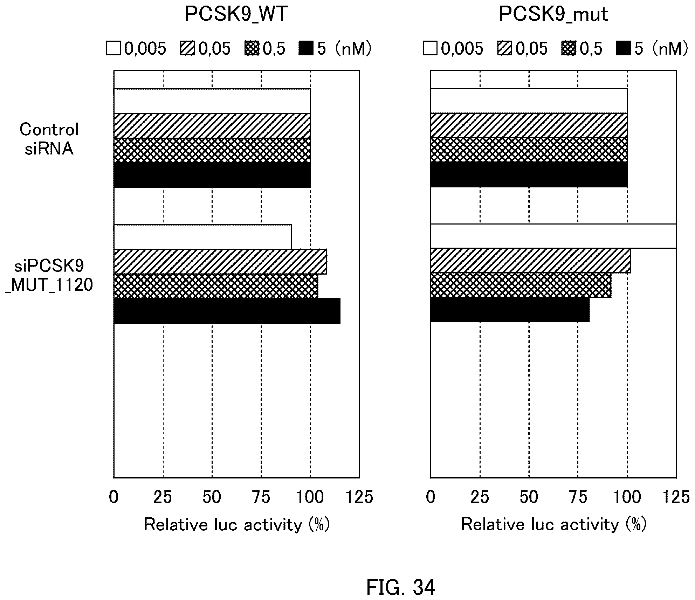

[Fig. 34] Fig. 34 shows graphs of the results on silencing abilities of siRNAs that target the *PCSK9* gene, in each of which position 11 corresponded to the position of the point mutation, the base at the 5'-end of the guide strand was changed from guanine to uracil, the base at the 5'-end of the passenger strand was changed from cytosine to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

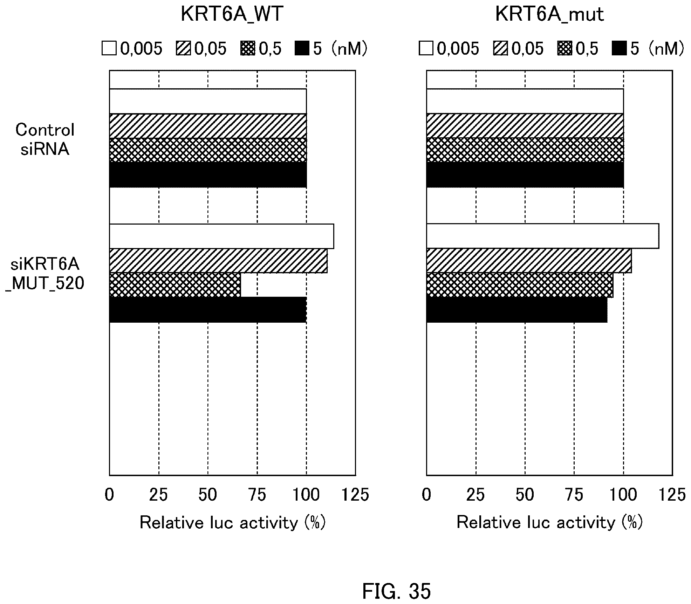

[Fig. 35] Fig. 35 shows graphs of the results on silencing abilities of siRNAs that target the *KRT6A* gene, in each of which position 11 corresponded to the position of the point mutation, the base at the 5'-end of the guide strand was changed from adenine to uracil, the base at the 5'-end of the passenger strand was changed from adenine to guanine, the group at the 2'-position of the pentose in each of ribonucleotides at positions 6-8 of the guide strand was replaced by OCH₃, and the base at position 6 of the guide strand was mismatched by changing it from guanine to cytosine, in an example of the present invention.

### Embodiments of the invention

Objects, characteristics, advantages, and ideas of the present invention are apparent to a person skilled in the art from the description of the present specification, and the person skilled in the art can easily reproduce the present invention from the description of the present specification. Modes for carrying out the invention, specific examples thereof and so forth, which are described below, provide preferable embodiments of the present invention. They are described for the purpose of illustration or explanation, and thus the present invention is not limited thereto. It is apparent to a person skilled in the art that various alterations and modifications can be made on the basis of the description of the present specification within the spirit and the scope of the present invention disclosed in the present specification.

The nucleotide sequences herein are indicated such that their 5'- and 3'-ends are located on the left and right sides, respectively, unless otherwise specified.

### == RNA molecules ==

An embodiment of the present invention is RNA molecules for use in RNA interference to target a mutant allele of a gene, the mutant allele having a point mutation relative to its corresponding wild-type allele. Any gene may be targeted as long as the RNA molecules described herein can be designed for it; however, the target is preferably an oncogene that can transform normal cells by a point mutation, a causative gene responsible for a genetic disease developed by a point mutation, or a causative gene responsible for a disease with an SNP linked to a causative mutation in its coding region. It is preferable that the percentage of the SNP's linking to the causative mutation in a genetic pool is 50% or more. It is more preferable that the percentage is 60% or more, 70% or more, 80% or more, or 90% or more. It is even more preferable that the percentage is 95% or more, 99% or more, or 99.5% or more.

Examples of the oncogene include the *ZMYM3, CTNNB1, SMARCA4, SMO,* and *AR* genes. Examples of the causative gene responsible for a genetic disease include the *DNM2, KRT14*, *IL4R, MAPT*, *MS4A2*, *PABPN1, SCNIA, APOB, F12, CLCN7, SCN8A, PCSK9, KRT6A,* and *RHO* genes. Examples of the causative gene responsible for a disease with an SNP include the *ATXN3* and *HTT* genes. Diseases that would be caused by these mutations are listed in Table 1.

The RNA molecules herein may consist of any number of nucleotides, and the number may be 13 or more and 100 or less, 13 or more and 50 or less, 13 or more and 28 or less, 15 or more and 25 or less, or 17 or more and 21 or less. More preferably, the number is 19 or more and 21 or less. One or more ribonucleotides may be each replaced by a deoxyribonucleotide, an artificial nucleic acid, or a nucleic acid analog such as inosine or morpholino. Such RNA molecules are herein called "chimeric NA molecules," and the RNA molecules are described as including chimeric NA molecules in the present disclosure.

In the RNA molecules, the base at position 5 or 6, counted from the base at the 5'-end of a nucleotide sequence complementary to that of a mutant allele, is mismatched with the base of the mutant allele. The RNA molecules have a nucleotide sequence complementary to that of the coding region of the mutant allele in the rest of the nucleotide sequence. Furthermore, the RNA molecules may have a sequence other than the nucleotide sequence complementary to that of the coding region of the mutant allele, such as a sequence complementary to the complementary nucleotide sequence, with which the molecules may be self-annealed to function as siRNA. An example of such a single-stranded RNA is Bonac nucleic acid. Alternatively, 1-3 nucleotide(s) may be added to its 3'-end, whose nucleotide sequences are not limited. If the base at the 5'-end of the complementary nucleotide sequence is not adenine or uracil, it may be replaced by adenine, uracil or thymine. If the base at the 3'-end of the complementary nucleotide sequence is not cytosine or guanine, it may be replaced by cytosine or guanine. These modifications enhance the gene expression suppression ability of the RNA molecules when they work as siRNA's guide strands. The RNA molecules may also contain chemical substances in addition to nucleic acids for delivery, to increase membrane permeability, or improve blood retention. For example, the RNA molecules may be conjugated to GalNAc or PEG. The RNA molecules may consist of a sequence other than the nucleotide sequence completely complementary to that of the coding region of the mutant allele except for the base at position 5 or 6. Notably, the RNA molecules have a nucleotide sequence complementary to that of the mutant allele except for the base at position 5 or 6, and their sequence complementarity is preferably 90% or more, more preferably 95% or more, yet more preferably 98% or more, and most preferably 100%. The base at position 5 or 6 may be any base, provided it is mismatched with that of the mutant allele. The base may be A, U, C, G, T, I, or any other artificial nucleic acid or nucleic acid analog as long as it differs from that in the corresponding position of the mutant allele.

Moreover, in the RNA molecules, the base at position 10 or 11, counted from the base at the 5'-end of the nucleotide sequence complementary to that of the mutant allele, is at the position of the point mutation and is identical to the base in the corresponding position of the mutant allele. In the case that the mutated base in the mutant allele is adenine, cytosine, guanine, or thymine, the base at position 10 or 11 of the RNA molecules is adenine, cytosine, guanine, or uracil (or thymine), respectively.

In the RNA molecules, the group at the 2'-position of the pentose in each of their nucleotides at positions 6-8 or positions 7 and 8, counted from the base at the 5'-end of the nucleotide sequence complementary to that of the mutant allele, is independently modified with (i.e., replaced by) OCH₃, halogen, or LNA. For example, RNA in which the group at the 2'-position of the pentose is replaced by -OCH₃ (hereinafter referred to as a "2'-O-methyl RNA") has a structure represented by the following general formula:

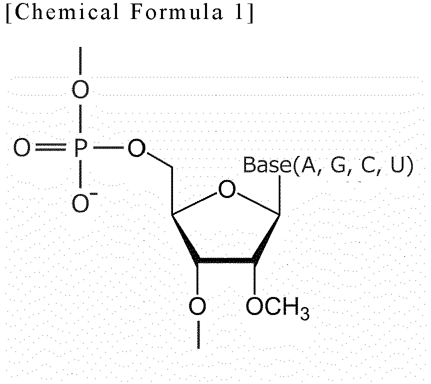

Any halogen may be used, but fluorine is preferred because of its small molecular size. For nucleotides at positions other than those mentioned, some or all of them may be modified; however, it is preferable that none of them is modified. Modifications of nucleotides are not particularly limited and exemplified that the group at the 2'-position of the pentose in the nucleotides is replaced by a group selected from the group consisting of H, OR, R, halogen, SH, SR, NH₂, NHR, NR₂, CN, COOR, and LNA, in which R is C₁-C₆ alkyl, alkenyl, alkynyl, or aryl; and halogen is F, Cl, Br, or I.

The IC50 of the RNA molecules for the target is preferably 1 nM or less, more preferably 500 pM or less, and yet more preferably 200 pM or less.

When the RNA molecules are used for RNA interference as a single strand, it is preferable that their 5'-end is phosphorylated or can be phosphorylated *in situ* or *in vivo.*

A method of designing the RNA molecules includes the following steps.

First, the step is performed in which a nucleotide sequence of a certain length containing a sequence complementary to that of a mutant allele is designed such that a mutated base in the mutant allele is placed at tenth or eleventh position, counted from the base at the 5'-end. The next step is to place a mismatched base at position 5 or 6, counted from the base at the 5'-end. Then, the group at the 2'-position of the pentose in each of nucleotides at positions 6-8 or positions 7 and 8, counted from the base at the 5'-end, is independently modified with OCH₃, halogen, or LNA. In the case that the base at the 5'-end of the complementary nucleotide sequence is not adenine or uracil, the step of replacing it by adenine or uracil or thymine may be performed. Likewise, in the case that the base at the 3'-end of the complementary nucleotide sequence is not cytosine or guanine, the step of replacing it by cytosine or guanine may be performed. Finally, 1-3 base(s) may be added to the 3'-end. In this way, a nucleotide sequence can be designed. A program for causing a computer to perform this design method may be made, and the program may be stored in a computer-readable recording medium. Nucleotides with a sequence designed in this manner can be chemically synthesized according to a routine method.

### == Double-stranded RNA molecules ==

An embodiment of the present invention is double-stranded RNA molecules in which one of the aforementioned RNA molecules (hereinafter referred to as a "first RNA molecule") serves as a guide strand, and a second RNA molecule with a sequence complementary to that of the first RNA molecule serves as a passenger strand. The second RNA molecule has a sequence complementary to that of the first RNA molecule and forms a duplex with the first RNA molecule under physiological conditions. Their sequence complementarity is preferably 90% or more, more preferably 95% or more, yet more preferably 98% or more, and most preferably 100%.

The passenger strand may have any length and may be considerably shorter than the first RNA molecule. For example, the length of the passenger strand may be equal to or less than half the length of the first RNA molecule. It is, however, preferable that they have the same length. When the passenger strand is shorter than the first RNA molecule, the latter has a single-stranded portion. This portion may be left single-stranded, or alternatively, a third RNA molecule complementary to the first RNA molecule may be annealed to the single-stranded portion. In the case that the second and third RNA molecules occupy the entire length of the first RNA molecule, the resulting double strand is identical to the one with a nick present on a single passenger strand to divide it into two.

The double-stranded RNA molecules may have two blunt ends, or alternatively, they may have an overhang at the 3'-end of either or both first and second RNA molecules serving as the guide and passenger strands, respectively. The overhang(s) may have any number of nucleotides but is/are preferably of 1-3 nucleotides long.

The double-stranded RNA molecule may be a double-stranded chimeric NA molecule in which 1-3, 4-6, 7-9, 10-12, 13-15, 16-18, 19-21, 22-24, or 25 or more ribonucleotides, or all ribonucleotides are each replaced by a deoxyribonucleotide, an artificial nucleic acid such as morpholine, or a nucleic acid analog such as glycol nucleic acid. The replaced positions are not limited.

The nucleotides in the passenger strand may be modified; however, it is preferable that they are not modified. Modifications of nucleotides are not particularly limited, and, for example, the group at the 2'-position of the pentose in the nucleotides may be replaced by a group selected from the group consisting of H, OR, R, halogen, SH, SR₁, NH₂, NHR, NR₂, CN, COOR, and LNA, in which R is C₁-C₆ alkyl, alkenyl, alkynyl, or aryl; and halogen is F, Cl, Br, or I.

Passenger strands can also be easily designed and easily produced using known techniques. A guide strand and a passenger strand may be linked to each other by a linker. The linker may be formed of any material, and examples include peptides and

### PEG.

### == RNA interference ==

An embodiment of the present invention is a method for performing RNA interference by which a mutant allele with a point mutation is targeted in cells containing the wild-type allele of a gene of interest and a mutant allele of the gene. This method includes the step of introducing the first RNA molecule that may include a chimeric NA molecule, or one of the aforementioned double-stranded RNA molecules that may include a double-stranded chimeric NA molecule, into the cells containing the wild-type allele and the mutant allele.

RNA interference can be readily performed using known techniques. The expression of a target can be reduced by introducing the first RNA molecule or the double-stranded RNA molecule into, for example, culture cells or a human or non-human individual organism expressing the target gene.

The use of the aforementioned first RNA molecules or double-stranded RNA molecules in RNA interference makes it possible to primarily suppress the expression of the mutant allele of the gene of interest, substantially not suppressing the expression of the wild-type allele. Here, the expression of the wild-type allele may be suppressed up to the level at which the wild-type allele is functional and a normal phenotype is exhibited. The expression of the mutant allele should be inhibited at least to the level at which the mutant allele is not functional and the abnormal phenotype is not exhibited. This enables, for example, cells to become functional normally without developing the phenotype due to a mutation even when the mutant allele carries a dominant mutation.

### == Agents ==

An embodiment of the present invention is a therapeutic agent for a patient with a disease or a prophylactic agent for a carrier of the disease, the patient or the carrier having wild-type and mutant alleles of a causative gene for the disease, the mutant allele of the causative gene having a point mutation and being responsible for the disease, wherein the therapeutic or prophylactic agent includes, as an active ingredient, any of the aforementioned RNA molecules including any one of the aforementioned chimeric NA molecules or any one of double-stranded RNA molecules including any one of the aforementioned double-stranded chimeric NA molecules. Carriers as used herein refer to individuals with a mutant allele of a causative gene for a disease who have not developed it and may develop it in the future. Prophylactic agents for carriers help prevent them from developing the disease due to the mutant allele of the causative gene.

Here, the cause of the disease may not be the point mutation but rather a different mutation and a certain percentage of patients or carriers of the disease have the point mutation. In the latter cases, the point mutation is preferably associated with the causative mutation. The aforementioned percentage is preferably 50% or more; more preferably, 60% or more, 70% or more, 80% or more, or 90% or more; and yet more preferably, 95% or more, 99% or more, or 99.5% or more, although not specifically limited. If the percentage is low, the patient or carrier may be examined to determine whether they have the point mutation before administering the agent. In these cases, healthy persons other than the patient or carrier preferably do not have the point mutation.

Examples of the former cases are genetic diseases and tumors that are caused by a point mutation. The genetic diseases are not limited as long as their development is caused by a point mutation, among which some examples are given in Table 1. Likewise, the tumors are not limited as long as they are caused by a point mutation in an oncogene, among which some examples are given in Table 1.

Examples of the latter include triplet repeat diseases. Triplet repeat diseases are known to be caused by 5-40 repeats and 36-3000 repeats of a triplet sequence such as CAG in healthy individuals and patients, respectively. For example, in the *ATXN3* mutant gene, which is a causative gene for Machado-Joseph disease, an SNP in which G immediately after a CAG repeat is mutated to C can be found. This mutation could be the target of the siRNA of the present disclosure. The triplet repeat diseases are not limited, among which some examples are given in Table 1.

Any method can be used for the administration of the agents disclosed herein; however, injection is preferable, and intravenous injection is more preferable. In such cases, in addition to the active ingredient, other ingredients such as pH adjusters, buffers, stabilizers, tonicity adjusting agents, or local anesthetics may be added to the therapeutic agents.

The dosage of the therapeutic agents is not limited and is selected as appropriate based on, for example, the efficacy of the ingredients contained, the mode of administration, the route of administration, the type of the disease, attributes of a subject (e.g., weight, age, medical conditions, and history of use of other medicaments), and the discretion of a physician in charge.

### == Selection methods ==

An embodiment of the present invention is a method for selecting RNA molecules, chimeric NA molecules, double-stranded RNA molecules, or double-stranded chimeric NA molecules for use in RNA interference to silence a target, the selection method including the steps of evaluating a gene-specific silencing ability of a plurality of the aforementioned RNA molecules, chimeric NA molecules, double-stranded RNA molecules, or double-stranded chimeric NA molecules by performing RNA interference *in vitro* using them; and selecting RNA molecules, chimeric NA molecules, double-stranded RNA molecules, or double-stranded chimeric NA molecules having at least a certain level of the gene-specific silencing ability.

In performing RNA interference *in vitro,* a wild-type allele and a mutant allele with a point mutation, of a gene are used as targets, and a molecule is selected which does not suppress the expression of the wild-type allele to a given level but suppresses the expression of the mutant allele to a level equal to or lower than a given level. By using this procedure, one or more molecules that suppress the expression of the mutant allele but do not suppress the expression of the wild-type allele can be obtained. Here, the given level may be any level, but is preferably 50%, more preferably 70%, and even more preferably 90%.

Methods for the assay using RNA interference *in vitro* are common knowledge in the art, and the selection of genes, selection of cells, and introduction of RNA molecules into cells, etc. are obvious to those skilled in the art.

### Examples

### (Method)

HeLa cells cultured in DMEM supplemented with 10% FBS were seeded at 1 × 10⁵ cells/mL on 24-well plates and co-transfected with each double-stranded siRNA with 100 ng of a reporter and 100 ng of plasmid (pGL3) as an internal standard, using 2 µL of lipofectamine 2000. The concentrations of the double-stranded siRNAs are shown in the figures. siGY441 was introduced as a control for siRNA. The cells were harvested after 24 hours, and firefly and Renilla luciferase activities were measured using a dual-luciferase reporter assay system (Promega). Renilla luciferase activity was normalized from the firefly luciferase activity. The results obtained for the double-stranded siRNAs are presented in graphs, relative to those for siGY441, which were set to 100%.

### Example 1

In this example, the K*-ras* gene was used as a target gene to be silenced.

### (Example 1-1)

This example shows that, by matching position 10 or 11 of each siRNA with the position of the point mutation in the A-mutant allele of the K*-ras* gene (c. 35G>A) (hereinafter, referred to as the "A-mutant allele"), the RNA molecules exhibit a higher specificity for silencing abilities to the A-mutant allele of the K*-ras* gene (35G>A) than to the wild-type allele of the K*-ras* gene (hereinafter, referred to as a "wild-type allele").

First, as reporters for examining gene silencing effects, DNAs with the same nucleotide sequences as the wild-type allele of the K*-ras* gene (wt) and the A-mutant allele of the K*-ras* gene (c. 35G>A) were chemically synthesized and inserted into the 3'-UTR of the luciferase gene in an expression vector (psiCHECK) to construct wild-type and A-mutant K reporters, respectively. The sequences of the segments incorporated into the vectors are indicated below.

Wild-type K reporter:
   5'-TGGTAGTTGGAGCTGGTGGCGTAGGCAAGAGTG-3' (SEQ ID NO. 4)
   3'-ACCATCAACCTCGACCACCGCATCCGTTCTCAC-5' (SEQ ID NO. 5)
A-mutant K reporter:
   5'-TGGTAGTTGGAGCTGATGGCGTAGGCAAGAGTG-3' (SEQ ID NO. 6)
   3'-ACCATCAACCTCGACTACCGCATCCGTTCTCAC-5' (SEQ ID NO. 7)

Next, double-stranded RNAs with the following sequences were chemically synthesized for siRNAs. The positions 9, 10, and 11 in siRNAs, K(35)9A, K(35)10A, and K(35)11A, respectively, correspond to the position of the point mutation in the A-mutant allele of the K*-ras* gene (c. 35G>A). In the following sequences, base pairs in the position corresponding to the position of the point mutation are enclosed in rectangles.

K(35)9A:
   5'- GUUGGAGCUGAUGGCGUAGTT-3' (SEQ ID NO. 8)
   3'-TTCAACCUCGACUACCGCAUC -5' (SEQ ID NO. 9)
K(35)10A:
   5'- UUGGAGCUGAUGGCGUAGGCA-3' (SEQ ID NO. 10)
   3'-UCAACCUCGACUACCGCAUCC -5' (SEQ ID NO. 11)
K(35)11A:
   5'- UGGAGCUGAUGGCGUAGGCAA-3' (SEQ ID NO. 12)
   3'-CAACCUCGACUACCGCAUCCG -5' (SEQ ID NO. 13)

Fig. 1 shows gene silencing effects of the siRNAs.

K(35)9A had a strong silencing effect on both A-mutant and wild-type alleles. K(35)10A and K(35)11A strongly suppressed the expression of the A-mutant allele more than that of the wild-type allele although their silencing effects were slightly reduced.

### (Example 1-2)

This example shows that the silencing abilities of the RNA molecule to the A-mutant allele become stronger and its specificities become much higher by, in addition to matching position 11 of an siRNA with the position of the point mutation in the A-mutant allele, changing the base at the 5'-end of the siRNA's guide strand from guanine to uracil and changing the base at the 5'-end of the passenger strand from uracil to guanine.

The wild-type and A-mutant K reporters were used as reporters for examining gene silencing effects. A double-stranded RNA with the following sequences was chemically synthesized for an siRNA, and K(35)11A was used as a control. In the following sequences, a base pair in the position corresponding to the position of the point mutation, and the pairs of the modified bases at the 5'-ends of the guide and passenger strands are enclosed in rectangles.

K(35)11Arev:
5'- GGGAGCUGAUGGCGUAGGAAA-3' (SEQ ID NO. 14)
3'-CACCCUCGACUACCGCAUCCU -5' (SEQ ID NO. 15)

Fig. 2 shows gene silencing effects of the siRNAs.

K(35)11A strongly suppressed the expression of the A-mutant allele more than that of the wild-type allele, whereas K(35)1 1Arev exerted a stronger silencing effect on both, with a stronger suppression of the expression of the A-mutant allele than that of the wild-type allele.

### (Example 1-3)

This example shows that silencing abilities of the RNA molecules to the A-mutant allele become stronger and their specificities become much higher by, in addition to matching position 11 of each siRNA with the position of the point mutation in the A-mutant allele, changing the base at the 5'-end of the siRNA's guide strand from guanine to uracil, and changing the base at the 5'-end of the passenger strand from uracil to guanine, replacing the group at 2'-position of the pentose in each of ribonucleotides at positions 6-8 of the guide strand by OCH₃.

The wild-type and A-mutant K reporters were used as reporters for examining gene silencing effects. Double-stranded RNAs with the following sequences were chemically synthesized for siRNAs, and K(35)11Arev was used as a control. In the following sequences, the base pairs in the position corresponding to the position of the point mutation, and the pairs of the modified bases at the 5'-ends of the guide and passenger strands are enclosed in rectangles. The nucleotides in which the group at the 2'-position of the pentose was replaced by OCH₃ are hatched.

K(35)11ArevOM(2-5) :
   5'- GGGAGCUGAUGGCGUAGGAAA-3' (SEQ ID NO. 16)
   3'-CACCCUCGACUACCGCAUCCU -5' (SEQ ID NO. 17)
K(35)11ArevOM(6-8) :
   5'- GGGAGCUGAUGGCGUAGGAAA-3' (SEQ ID NO. 18)
   3'-CACCCUCGACUACCGCAUCCU -5' (SEQ ID NO. 19)

Fig. 3 shows gene silencing effects of the siRNAs.

K(35)11Arev strongly suppressed the expression of the A-mutant allele more than that of the wild-type allele, whereas K(35)11ArevOM(6-8) exerted a stronger silencing effect on both, with a stronger suppression of the expression of the A-mutant allele than that of the wild-type allele. Another control, K(35)11ArevOM(2-5) in which the group at the 2'-position of the pentose in each of ribonucleotides at positions 2-5 of the guide strand was replaced by OCH₃ exerted considerably weak silencing effect on both.

### (Example 1-4)

This example shows that silencing abilities of the RNA molecules to the wild-type allele become weaker and, as a result, their specificities for the A-mutant allele become much higher by, in addition to matching position 11 of each siRNA with the position of the point mutation in the A-mutant allele, changing the base at the 5'-end of the siRNA's guide strand from guanine to uracil, and changing the base at the 5'-end of the passenger strand from uracil to guanine, mismatching the base at position 5 or 6 of the guide strand with that of the A-mutant allele,.

The wild-type and A-mutant K reporters were used as reporters for examining gene silencing effects. Double-stranded RNAs with the following sequences with a mismatched base at one of positions 3-7 based on K(35)11Arev were chemically synthesized for siRNAs. K(35)11Arev was used as a control. In the following sequences, the base pairs in the position corresponding to the position of the point mutation, the pairs of the modified bases at the 5'-ends of the guide and passenger strands, and base pairs with the mismatched base are enclosed in rectangles.

K(35)11ArevM3:
   5'- GGGAGCUGAUGGCGUACGAAA-3' (SEQ ID NO. 20)
   3'-CACCCUCGACUACCGCAUGCU -5' (SEQ ID NO. 21)
K(35)11ArevM4:
   5'- GGGAGCUGAUGGCGUUGGAAA-3' (SEQ ID NO. 22)
   3'-CACCCUCGACUACCGCAACCU -5 (SEQ ID NO. 23)
K(35)11ArevM5:
   5'- GGGAGCUGAUGGCGAAGGAAA-3' (SEQ ID NO. 24)
   3'-CACCCUCGACUACCGCUUCCU -5' (SEQ ID NO. 25)
K(35)11ArevM6:
   5'- GGGAGCUGAUGGCCUAGGAAA-3' (SEQ ID NO. 26)
   3'-CACCCUCGACUACCGGAUCCU -5' (SEQ ID NO. 27)
K(35)11ArevM7:
   5'- GGGAGCUGAUGGGGUAGGAAA-3' (SEQ ID NO. 28)
   3'-CACCCUCGACUACCCCAUCCU -5' (SEQ ID NO. 29)

Fig. 4 shows gene silencing effects of the siRNAs.

K(35)11Arev strongly suppressed the expression of the A-mutant allele more than that of the wild-type allele, whereas RNA molecules K(35)11ArevM5 and K(35)11ArevM6 exhibited significantly weak silencing abilities to the wild-type allele and, as a result, much higher specificities for the A-mutant allele.

### (Example 1-5)

This example shows that specificities of the RNA molecules for the A-mutant allele become much higher by, in addition to matching position 11 of each siRNA with the position of the point mutation in the A-mutant allele, changing the base at the 5'-end of the siRNA's guide strand from guanine to uracil, and changing the base at the 5'-end of the passenger strand from uracil to guanine, replacing the group at the 2'-position of the pentose in each of ribonucleotides at positions 6-8 of the guide strand by OCH₃, and mismatching the base at position 5 or 6 of the guide strand with that of the A-mutant allele.

The wild-type and A-mutant K reporters were used as reporters for examining gene silencing effects. Double-stranded RNAs with the following sequences with a mismatched base at one of positions 3-7 based on K(35)11Arev were chemically synthesized for siRNAs. K(35)11Arev was used as a control. In the following sequences, the base pairs in the position corresponding to the position of the point mutation, the pairs of the modified bases at the 5'-ends of the guide and passenger strands, and base pairs with the mismatched base are enclosed in rectangles. The nucleotides in which the group at the 2'-position of the pentose was replaced by OCH₃ are hatched.

K(35)11ArevOM(6-8)M3:
   5'- GGGAGCUGAUGGCGUACGAAA-3' (SEQ ID NO. 30)
   3'-CACCCUCGACUACCGCAUGCU -5 (SEQ ID NO. 31)
K(35)11ArevOM(6-8)M4:
   5'- GGGAGCUGAUGGCGUUGGAAA-3 (SEQ ID NO. 32)
   3'-CACCCUCGACUACCGCAACCU -5 (SEQ ID NO. 33)
K(35)11ArevOM(6-8)M5:
   5'- GGGAGCUGAGGCGAAGGAAA-3 (SEQ ID NO. 34)
   3'-CACCCUCGACUACCGCUUCCU -5 (SEQ ID NO. 35)
K(35)11ArevOM(6-8)M6:
   5'- GGGAGCUGAUGGCCUAGGAAA-3' (SEQ ID NO. 36)
   3'-CACCCUCGACUACCGGAUCCU -5' (SEQ ID NO. 37)
K(35)11ArevOM(6-8)M7:
   5'- GGGAGCUGAUGGGGUAGGAAA-3' (SEQ ID NO. 38)
   3'-CACCCUCGACUACCCCAUCCU -5' (SEQ ID NO. 39)

Fig. 5 shows gene silencing effects of the siRNAs.

K(35)11ArevOM(6-8)M5 and K(35)11ArevOM(6-8)M6 exhibited very weak silencing abilities to the wild-type allele and, as a result, their specificities for the A-mutant allele became much higher.

### (Example 1-6)

This example shows that siRNAs specific for the A-mutant allele exhibit weak silencing abilities not only to the wild-type allele, but also to the T-mutant allele of the K*-ras* gene (c. 35G>T) and the C-mutant allele of the K*-ras* gene (c. 35 G>C).

K*-ras* reporters indicated below, i.e., the wild-type K reporter, the A-mutant K reporter, a T-mutant reporter for the K*-ras* gene (c. 35G>T) (hereinafter, referred to as the "T-mutant K reporter"), and a C-mutant reporter for the K*-ras* gene (c. 35 G>C) (hereinafter, referred to as the "C-mutant K reporter"), were used as reporters for examining gene silencing effects. K(35)11ArevOM(6-8)M5 and K(35)11ArevOM(6-8)M6 were used as siRNAs, and K(35)11Arev was used as a control. In the following sequences, the base pairs in the position corresponding to the position of the point mutation are enclosed in rectangles.

T-mutant K reporter:
   5'-TGGTAGTTGGAGCTGTTGGCGTAGGCAAGAGTG-3' (SEQ ID NO. 40)
   3'-ACCATCAACCTCGACAACCGCATCCGTTCTCAC-5' (SEQ ID NO. 41)
C-mutant K reporter:
   5'-TGGTAGTTGGAGCTGCTGGCGTAGGCAAGAGTG-3' (SEQ ID NO. 42)
   3'-ACCATCAACCTCGACGACCGCATCCGTTCTCAC-5' (SEQ ID NO. 43)

Fig. 6 shows gene silencing effects on the reporters.

All siRNAs had the strongest silencing effect on the A-mutant K reporter; especially K(35)11ArevOM(6-8)M5 and K(35)11ArevOM(6-8)M6 had weak silencing effects on the T-mutant and C-mutant K reporters.

### (Example 1-7)

This example shows that siRNAs specific for the T-mutant allele exhibit weak silencing abilities to the A-mutant and C-mutant alleles in addition to the wild-type allele.

K*-ras* reporters, i.e., the wild-type, A-mutant, T-mutant, and C-mutant K reporters, were used as reporters for examining gene silencing effects. K(35)11TrevOM(6-8)M5 and K(35)11TrevOM(6-8)M6 were used as siRNAs, and K(3 5) 11 Trev was used as a control. In the following sequences, the base pairs in the position corresponding to the position of the point mutation, the pairs of the modified bases at the 5'-ends of the guide and passenger strands, and the base pairs with the mismatched base are enclosed in rectangles. The nucleotides in which the group at 2'-position of the pentose was replaced by OCH₃ are hatched.

K(35)llTrev:
   5'- GGGAGCUGUUGGCGUAGGAAA-3' (SEQ ID NO. 44)
   3'-CACCCUCGACAACCGCAUCCU -5' (SEQ ID NO. 45)
K(35)llTrevOM(6-8)M5:
   5'- GGGAGCUGUUGGCGAAGGAAA-3' (SEQ ID NO. 46)
   3'-CACCCUCGACAACCGCUUCCU -5' (SEQ ID NO. 47)
K(35)llTrevOM(6-8)M6:
   5'- GGGAGCUGUUGGCCUAGGAAA-3' (SEQ ID NO. 48)
   3'-CACCCUCGACAACCGGAUCCU -5' (SEQ ID NO. 49)

Fig. 7 shows gene silencing effects on the reporters.

All siRNAs had the strongest silencing effect on the T-mutant K reporter; especially K(35)11TrevOM(6-8)M5 and K(35)11TrevOM(6-8)M6 had weak silencing effects on the T-mutant and C-mutant K reporters.

### (Example 1-8)

This example shows that siRNAs specific for the C-mutant allele exhibit weak silencing abilities to the A-mutant and T-mutant alleles in addition to the wild-type allele.

K*-ras* reporters, i.e., the wild-type, A-mutant, T-mutant, and C-mutant K reporters were used as reporters for examining gene silencing effects. K(35)11CrevOM(6-8)M5 and K(35)11CrevOM(6-8)M6 were used as siRNAs, and K(35)11Crev was used as a control. In the following sequences, the base pairs in the position corresponding to the position of the point mutation, the pairs of the modified bases at the 5'-ends of the guide and passenger strands, and the base pairs with the mismatched base are enclosed in rectangles. The nucleotides in which the group at the 2'-position of the pentose was replaced by OCH₃ are hatched.

K(35)11Crev:
   5'- GGGAGCUGCUGGCGUAGGAAA-3' (SEQ ID NO. 50)
   3'-CACCCUCGACGACCGCAUCCU -5' (SEQ ID NO. 51)
K(35)llCrevOM(6-8)M5:
   5'- GGGAGCUGGGCGAAGGAAA-3' (SEQ ID NO. 52)
   3'-CACCCUCGACGACCGCUUCCU -5' (SEQ ID NO. 53)
K(35)llCrevOM(6-8)M6:
   5'- GGGAGCUGCUGGCCUAGGAAA-3' (SEQ ID NO. 54)
   3'-CACCCUCGACGACCGGAUCCU -5' (SEQ ID NO. 55)

Fig. 8 shows gene silencing effects on the reporters.

All siRNAs had the strongest silencing effect on the C-mutant K reporter; especially K(35)11CrevOM(6-8) M5 and K(35)11CrevOM(6-8)M6 had weak silencing effect on the A-mutant and T-mutant K reporters.

### Example 2

In this example, the N*-ras* gene was chosen as a target gene to be silenced.

### (Example 2-1)

This example, targeting the point mutation in nt 35 of N*-ras* cDNA, shows that siRNAs suppress the expression of the A-mutant N35 allele more specifically than that of the wild-type allele of the N*-ras* (wt) gene (hereinafter, referred to as the "wild-type N allele") by matching position 11 of each siRNA with the position of the point mutation in the A-mutant allele of the N*-ras* gene (c. 35G>A) (hereinafter, referred to as the "A-mutant N35 allele"), changing the base at the 5'-end of the guide strand of the siRNA from cytosine to uracil, changing the base at the 5'-end of the passenger strand from adenine to guanine, replacing the group at the 2'-position of the pentose in each of ribonucleotides at positions 6-8 of the guide strand by OCH₃, and mismatching the base at position 5 of the guide strand with that of the A-mutant N35 allele.

First, as reporters for examining gene silencing effects, DNAs with the same nucleotide sequences as the wild-type N allele and the A-mutant N35 allele were inserted into the 3'-UTR of the luciferase gene in an expression vector (psiCHECK) to construct wild-type N35 and A-mutant N reporters, respectively. The sequences of the segments chemically synthesized and incorporated into the vectors are indicated below. In the following sequences, the base pairs in the position corresponding to the position of the point mutation are enclosed in rectangles.

Wild-type N35 reporter:
   5'-ACTGGTGGTGGTTGGAGCAGGTGGTGTTGGGAAAAGCGCA-3' (SEQ ID NO. 56)
   3'-TGACCACCACCAACCTCGACCACCACAACCCTTTTCGCGT-5' (SEQ ID NO. 57)
A-mutant N35 reporter:
   5'-ACTGGTGGTGGTTGGAGCAGATGGTGTTGGGAAAAGCGCA-3' (SEQ ID NO. 58)
   3'-TGACCACCACCAACCTCGACTACCACAACCCTTTTCGCGT-5' (SEQ ID NO. 59)

Double-stranded RNAs with the following sequences were chemically synthesized for siRNAs. N(35)11G has a sequence complementary to that of the wild-type N allele. N(35)11A is an siRNA in which position 11 corresponded to the position of the point mutation in the A-mutant N35 allele. N(35)11ArevOM(6-8)M5 is an siRNA in which position 11 corresponded to the position of the point mutation in the A-mutant N35 allele, the base at the 5'-end of the guide strand was changed from cytosine to uracil, the base at the 5'-end of the passenger strand was changed from uracil to cytosine, the group at the 2'-position of the pentose in each of ribonucleotides at positions 6-8 of the guide strand was replaced by OCH₃, and the base at position 5 of the siRNA's guide strand was mismatched with that of the A-mutant N35 allele. In the following sequences, the base pairs in the position corresponding to the position of the point mutation, the pairs of the modified bases at the 5'-ends of the guide and passenger strands, and the base pairs with the mismatched base are enclosed in rectangles. The nucleotides in which the group at the 2'-position of the pentose was replaced by OCH₃ are hatched.

N(35)11G:
   5'- UGGAGCAGGUGGUGUUGGGAA-3' (SEQ ID NO. 60)
   3'-CAACCUCGUCCACCACAACCC -5' (SEQ ID NO. 61)
N(35)11A:
   5'- UGGAGCAGAUGGUGUUGGGAA-3' (SEQ ID NO. 62)
   3'-CAACCUCGUCUACCACAACCC -5' (SEQ ID NO. 63)
N(35)11ArevOM(6-8)M5:
   5'- CGGAGCAGAUGGUGAUGGAAA-3' (SEQ ID NO. 64)
   3'-CAGCCUCGUCUACCACUACCU -5' (SEQ ID NO. 65)

Fig. 9 shows gene silencing effects of the siRNAs.

N(35)11G effectively suppressed the expression of the wild-type N allele more than that of the A-mutant N35 allele. In contrast, N(35)11A effectively suppressed the expression of the A-mutant N35 allele more than that of the wild-type N allele. N(35)11ArevOM(6-8)M5 had very weak silencing abilities to the wild-type allele and strong silencing abilities to the A-mutant N35 allele; as a result, specificity for the A-mutant N35 allele was increased.

### (Example 2-2)

This example, targeting the point mutation in nt 182 of N*-ras* cDNA shows that siRNAs suppress the expression of the G-mutant N182 allele more specifically than that of the wild-type allele of the N*-ras* (wt) gene (hereinafter, referred to as the "wild-type N allele") by matching position 11 of each siRNA with the position of the point mutation in the G-mutant allele of the N*-ras* gene (c. 182A>G) (hereinafter, referred to as the "G-mutant N182 allele"), changing the base at the 5'-end of the guide strand of the siRNA from guanine to uracil, changing the base at the 5'-end of the passenger strand from adenine to guanine, replacing the group at the 2'-position of the pentose in each of ribonucleotides at positions 6-8 of the guide strand by OCH₃, and mismatching the base at position 5 of the guide strand with that of the G-mutant N182 allele.

First, as reporters for examining gene silencing effects, DNAs with the same nucleotide sequence as the G-mutant N182 allele were inserted into the 3'-UTR of the luciferase gene in an expression vector (psiCHECK) to construct a G-mutant N182 reporter. The sequences of the segments chemically synthesized and incorporated into the vector are indicated below.

Wild-type N182 reporter:
   5'-CATACTGGATACAGCTGGACAAGAAGAGTACAGTGCCA-3' (SEQ ID NO. 66)
   3'-GTATGACCTATGTCGACCTGTTCTCTTCTCATGTCACGGT-5' (SEQ ID NO. 67)
G-mutant N182 reporter:
   5'-CATACTGGATACAGCTGGACGAGAAGAGTACAGTGCCA-3' (SEQ ID NO. 68)
   3'-GTATGACCTATGTCGACCTGCTCTTCTCATGTCACGGT-5' (SEQ ID NO. 69)

Double-stranded RNAs with the following sequences were chemically synthesized for siRNAs. N(182)11A has a sequence complementary to that of the wild-type N allele. N(182)11G is an siRNA in which position 11 corresponded to the position of the point mutation in the G-mutant N182 allele. N(182)11GrevOM(6-8)M5 is an siRNA in which position 11 corresponded to the position of the point mutation in the G-mutant N182 allele, the base at the 5'-end of the guide strand was changed from guanine to uracil, the base at the 5'-end of the passenger strand was changed from adenine to guanine, the group at the 2'-position of the pentose in each of ribonucleotides at positions 6-8 of the guide strand was replaced by OCH₃, and the base at position 5 of the siRNA's guide strand was mismatched with that of the G-mutant N182 allele. In the following sequences, the base pairs in the position corresponding to the position of the point mutation, the pairs of the modified bases at the 5'-ends of the guide and passenger strands, and the base pairs with the mismatched base are enclosed in rectangles. The nucleotides in which the group at the 2'-position of the pentose was replaced by OCH₃ are hatched.

N(182)11A:
   5'- AGCUGGACAAGAAGAGUACAG-3' (SEQ ID NO. 70)
   3'-UGUCGACCUGUUCUUCUCAUG -5' (SEQ ID NO. 71)
N(182)11G:
   5'- AGCUGGACGAGAAGAGUACAG-3' (SEQ ID NO. 72)
   3'-UGUCGACCUGCUCUUCUCAUG -5' (SEQ ID NO. 73)
N(182)11GrevOM(6-8)M5:
   5'- GGCUGGACGAGAAGUGUAAAG-3' (SEQ ID NO. 74)
   3'-UGCCGACCUGCUCUUCACAUU -5' (SEQ ID NO. 75)

Fig. 10 shows gene silencing effects of the siRNAs.

N(182)11A effectively suppressed the expression of the wild-type N182 allele more than that of the G-mutant N182 allele. In contrast, N(182)11G effectively suppressed the expression of the A-mutant N182 allele more than that of the wild-type N182 allele. N(182)11ArevOM(6-8)M5 lost silencing abilities to the wild-type allele and exhibited slightly reduced silencing abilities to the G-mutant N182 allele, resulting in a higher specificity for the G-mutant N182 allele.

### Example 3

In this example, various genes listed in Table 2 were used as the target genes to be silenced to show that siRNAs designed according to the method disclosed herein suppress the expression of the mutant alleles but do not suppress the expression of the wild-type alleles.

First, as reporters for examining gene silencing effects, DNAs with the same nucleotide sequences as wild-type (wt) and mutant alleles were chemically synthesized and inserted into the 3'-UTR of the luciferase gene in an expression vector (psiCHECK) to construct wild-type and mutant reporters, respectively. The sequences of the segments incorporated into the vectors are indicated in Table 2.

In the nucleotide sequences in the tables, lowercase letters denote sequences for ligating to a vector, and capital letters denote sequences derived from a gene. The parentheses in the K*-ras* gene indicate the type of mutation, the parentheses in the *HTT* gene indicate the position in the genome, and the parentheses in other genes indicate a place counted from the translation start site (i.e., A in the start codon ATG). "P" stands for a passenger strand, and "G" stands for a guide strand.

**[Table 2]**

| Gene Name (Position of mutation) | Function | Strand | Name | Sequence | Seq. ID No. |
|---|---|---|---|---|---|
| HTT (rs363125) | Wild-type reporter | S | HTT_363125_WT_s | tcgagGTTAAGAGATCCCCACAGTACTTCAACGCTAGAAGAACACAg | 76 |
| | | AS | HTT_363125_WT_as | aattcTGTGTTCTTCTAGCGTTGAAGTACTGTGGGGATCTCTTAACc | 77 |
| | Mutant reporter | S | HTT_363125_SNP_s | tcgagGTTAAGAGATCCCCACAGTAATTCAACGCTAGAAGAACACAg | 78 |
| | | AS | HTT_363125_SNP_as | aattcTGTGTTCTTCTAGCGTTGAATTACTGTGGGGATCTCTTAACc | 79 |
| HTT (rs362307) | Wild-type reporter | S | HTT_362307_WT_s | tcgagCCGGAGCCTTTGGAAGTCTGCGCCCTTGTGCCCTGCCTCCAg | 80 |
| | | AS | HTT_362307_WT_as | aattcTGGAGGCAGGGCACAAGGGCGCAGACTTCCAAAGGCTCCGGc | 81 |
| | Mutant reporter | S | HTT_362307_SNP_s | tcgagCCGGAGCCTTTGGAAGTCTGTGCCCTTGTGCCCTGCCTCCAg | 82 |
| | | AS | HTT_362307_SNP_as | aattcTGGAGGCAGGGCACAAGGGCACAGACTTCCAAAGGCTCCGGc | 83 |
| HTT (rs362331) | Wild-type reporter | S | HTT_362331_WT_s | tcgagCCCACGCCTGCTCCCTCATCTACTGTGTGCACTTCATCCTGg | 84 |
| | | AS | HTT_362331_WT_as | aattcCAGGATGAAGTGCACACAGTAGATGAGGGAGCAGGCGTGGGc | 85 |
| | Mutant reporter | S | HTT_362331_SNP_s | tcgagCCCACGCCTGCTCCCTCATCCACTGTGTGCACTTCATCCTGg | 86 |
| | | AS | HTT_362331_SNP_as | aattcCAGGATGAAGTGCACACAGTGGATGAGGGAGCAGGCGTGGGc | 87 |
| ATXN3 | Wild-type reporter | S | ATXN3_WT_s | tcgagAGCAGCAGCAGCAGCAGCAGGGGGACCTATCAGGACAGAGTg | 88 |
| | | AS | ATXN3_WT_as | aattcACTCTGTCCTGATAGGTCCCCCTGCTGCTGCTGCTGCTGCTc | 89 |
| | Mutant reporter | S | ATXN3_SNP_s | tcgagAGCAGCAGCAGCAGCAGCAGCGGGACCTATCAGGACAGAGTg | 90 |
| | | AS | ATXN3_SNP_as | aattcACTCTGTCCTGATAGGTCCCGCTGCTGCTGCTGCTGCTGCTc | 91 |
| ZMYM3 | Wild-type reporter | S | ZMYM3_WT_Sense | tcgagTGCTGGAGACCATCCACTGGCGTGGGCAGATCCGTCATTTCg | 92 |
| | | AS | ZMYM3_WT_Anti-sense | aattcGAAATGACGGATCTGCCCACGCCAGTGGATGGTCTCCAGCAc | 93 |
| | Mutant reporter | S | ZMYM3_2206_Sense | tcgagTGCTGGAGACCATCCACTGGTGTGGGCAGATCCGTCATTTCg | 94 |
| | | AS | ZMYM3_2206_Anti-sense | aattcGAAATGACGGATCTGCCCACAGCAGTGGATGGTCTCCAGCAc | 95 |
| CTNNB1 | Wild-type reporter | S | CTNNB1_WT_Sense | tcgagGAATCCATTCTGGTGCCACTACCACAGCTCCTTCTCTGAGTg | 96 |
| | | AS | CTNNB1_WT_Anti-sense | aattcACTCAGAGAAGGAGCTGTGGTAGTGGCACCAGAATGGATTCc | 97 |
| | Mutant reporter | S | CTNNB1_121_Sense | tcgagGAATCCATTCTGGTGCCACTGCCACAGCTCCTTCTCTGAGTg | 98 |
| | | AS | CTNNB1_121_Anti-sense | aattcACTCAGAGAAGGAGCTGTGGCAGTGGCACCAGAATGGATTCc | 99 |
| SMARCA4 | Wild-type reporter | S | SMARCA4_WT_Sense | tcgagACCCCGCCGCCTGCTGCTGACGGGCACACCGCTGCAGAACAg | 100 |
| | | AS | SMARCA4_WT_Anti-sense | aattcTGTTCTGCAGCGGTGTGCCCGTCAGCAGCAGGCGGCGGGGTc | 101 |
| | Mutant reporter | S | SMARCA4_2729_Sense | tcgagACCCCGCCGCCTGCTGCTGATGGGCACACCGCTGCAGAACAg | 102 |
| | | AS | SMARCA4_2729_Anti-sense | aattcTGTTCTGCAGCGGTGTGCCCATCAGCAGCAGGCGGCGGGGTc | 103 |
| SMO | Wild-type reporter | S | SMO_WT_Sense | tcgagTGGCCCCAATCGGCCTGGTGCTCATCGTGGGAGGCTACTTCg | 104 |
| | | AS | SMO_WT_Anti-sense | aattcGAAGTAGCCTCCCACGATGAGCACCAGGCCGATTGGGGCCAc | 105 |
| | Mutant reporter | S | SMO_1234_Sense | tcgagTGGCCCCAATCGGCCTGGTGTTCATCGTGGGAGGCTACTTCg | 106 |
| | | AS | SMO_1234_Anti-sense | aattcGAAGTAGCCTCCCACGATGAACACCAGGCCGATTGGGGCCAc | 107 |
| AR | Wild-type reporter | S | AR WT Sense | tcgagCAAGGCCTTGCCTGGCTTCCGCAACTTACACGTGGACGACCg | 108 |
| | | AS | AR WT Anti-sense | aattcGGTCGTCCACGTGTAAGTTGCGGAAGCCAGGCAAGGCCTTGc | 109 |
| | Mutant reporter | S | AR 2180 Sense | tcgagCAAGGCCTTGCCTGGCTTCCTCAACTTACACGTGGACGACCg | 110 |
| | | AS | AR 2180 Anti-sense | aattcGGTCGTCCACGTGTAAGTTGAGGAAGCCAGGCAAGGCCTTGc | 111 |
| DNM2 | Wild-type reporter | S | DNM2 WT Sense | tcgagGAATCAATCGCATCTTCCACGAGCGGTTCCCATTTGAGCTGg | 112 |
| | | AS | DNM2 WT Anti-sense | aattcCAGCTCAAATGGGAACCGCTCGTGGAAGATGCGATTGATTCc | 113 |
| | Mutant reporter | S | DNM2 1102 Sense | tcgagGAATCAATCGCATCTTCCACAAGCGGTTCCCATTTGAGCTGg | 114 |
| | | AS | DNM2 1102 Anti-sense | aattcCAGCTCAAATGGGAACCGCTTGTGGAAGATGCGATTGATTCc | 115 |
| KRT14 | Wild-type reporter | S | KRT14 WT Sense | tcgagTCAAGACCCTCAACAACAAGTTTGCCTCCTTCATCGACAAGg | 116 |
| | | AS | KRT14 WT Anti-sense | aattcCTTGTCGATGAAGGAGGCAAACTTGTTGTTGAGGGTCTTGAc | 117 |
| | Mutant reporter | S | KRT14 520 Sense | tcgagTCAAGACCCTCAACAACAAGGTTGCCTCCTTCATCGACAAGg | 118 |
| | | AS | KRT14 520 Anti-sense | aattcCTTGTCGATGAAGGAGGCAACCTTGTTGTTGAGGGTCTTGAc | 119 |
| IL4R (223) | Wild-type reporter | S | IL4R WT Sense | tcgagTCTCCGAAGCCCACACGTGTATCCCTGAGAACAACGGAGGCg | 120 |
| | | AS | IL4R WT Anti-sense | aattcGCCTCCGTTGTTCTCAGGGATACACGTGTGGGCTTCGGAGAc | 121 |
| | Mutant reporter | S | IL4R_223_Sense | tcgagTCTCCGAAGCCCACACGTGTGTCCCTGAGAACAACGGAGGCg | 122 |
| | | AS | IL4R 223 Anti-sense | aattcGCCTCCGTTGTTCTCAGGGACACACGTGTGGGCTTCGGAGAc | 123 |
| IL4R (1507) | Wild-type reporter | S | IL4R_WT_Sense | tcgagCTTACCGCAGCTTCAGCAACTCCCTGAGCCAGTCACCGTGTg | 124 |
| | | AS | IL4R_WT_Anti-sense | aattcACACGGTGACTGGCTCAGGGAGTTGCTGAAGCTGCGGTAAGc | 125 |
| | Mutant reporter | S | IL4R_1507_Sense | tcgagCTTACCGCAGCTTCAGCAACCCCCTGAGCCAGTCACCGTGTg | 126 |
| | | AS | IL4R_1507_Anti-sense | aattcACACGGTGACTGGCTCAGGGGGTTGCTGAAGCTGCGGTAAGc | 127 |
| MAPT (1853) | Wild-type reporter | S | MAPT WT Sense | tcgagGGATAATATCAAACACGTCCCGGGAGGCGGCAGTGTGCAAAg | 128 |
| | | AS | MAPT WT Anti-sense | aattcTTTGCACACTGCCGCCTCCCGGGACGTGTTTGATATTATCCc | 129 |
| | Mutant reporter | S | MAPT 1853 Sense | tcgagGGATAATATCAAACACGTCCTGGGAGGCGGCAGTGTGCAAAg | 130 |
| | | AS | MAPT 1853 Anti-sense | aattcTTTGCACACTGCCGCCTCCCAGGACGTGTTTGATATTATCCc | 131 |
| MS4A2 | Wild-type reporter | S | MS4A2 WT Sense | tcgagTGAGTTGGAAGACCCAGGGGAAATGTCTCCTCCCATTGATTg | 132 |
| | | AS | MS4A2 WT Anti-sense | aattcAATCAATGGGAGGAGACATTTCCCCTGGGTCTTCCAACTCAc | 133 |
| | Mutant reporter | S | MS4A2 710 Sense | tcgagTGAGTTGGAAGACCCAGGGGGAATGTCTCCTCCCATTGATTg | 134 |
| | | AS | MS4A2 710 Anti-sense | aattcAATCAATGGGAGGAGACATTCCCCCTGGGTCTTCCAACTCAc | 135 |
| PABPN1 | Wild-type reporter | S | PABPN1 WT Sense | tcgagGGCGGCGGCAGCAGCAGCGGGGGCTGCGGGCGGTCGGGGCTg | 136 |
| | | AS | PABPN1 WT Anti-sense | aattcAGCCCCGACCGCCCGCAGCCCCCGCTGCTGCTGCCGCCGCCc | 137 |
| | Mutant reporter | S | PABPN1_35_Sense | tcgagGGCGGCGGCAGCAGCAGCGGCGGCTGCGGGCGGTCGGGGCTg | 138 |
| | | AS | PABPN1_35_Anti-sense | aattcAGCCCCGACCGCCCGCAGCCGCCGCTGCTGCTGCCGCCGCCc | 139 |
| RHO (68) | Wild-type reporter | S | RHO WT Sense | tcgagGACGGGTGTGGTACGCAGCCCCTTCGAGTACCCACAGTACTg | 140 |
| | | AS | RHO WT Anti-sense | aattcAGTACTGTGGGTACTCGAAGGGGCTGCGTACCACACCCGTCc | 141 |
| | Mutant reporter | S | RHO 68 Sense | tcgagGACGGGTGTGGTACGCAGCCACTTCGAGTACCCACAGTACTg | 142 |
| | | AS | RHO_68_Anti-sense | aattcAGTACTGTGGGTACTCGAAGTGGCTGCGTACCACACCCGTCc | 140 |
| RHO (1039) | Wild-type reporter | S | RHO WT Sense | tcgagCGGAGACGAGCCAGGTGGCCCCGGCCTAAGACCTGCCTAGGg | 141 |
| | | AS | RHO WT Anti-sense | aattcCCTAGGCAGGTCTTAGGCCGGGGCCACCTGGCTCGTCTCCGc | 143 |
| | Mutant reporter | S | RHO 1039 Sense | tcgagCGGAGACGAGCCAGGTGGCCTCGGCCTAAGACCTGCCTAGGg | 144 |
| | | AS | RHO 1039 Anti-sense | aattcCCTAGGCAGGTCTTAGGCCGAGGCCACCTGGCTCGTCTCCGc | 145 |
| SCNIA | Wild-type reporter | S | SCN1A WT Sense | tcgagTGAGAACATTCAGAGTTCTCCGAGCATTGAAGACGATTTCAg | 146 |
| | | AS | SCN1A WT Anti-sense | aattcTGAAATCGTCTTCAATGCTCGGAGAACTCTGAATGTTCTCAc | 147 |
| | Mutant reporter | S | SCN1A 664 Sense | tcgagTGAGAACATTCAGAGTTCTCTGAGCATTGAAGACGATTTCAg | 148 |
| | | AS | SCN1A 664 Anti-sense | aattcTGAAATCGTCTTCAATGCTCAGAGAACTCTGAATGTTCTCAc | 149 |
| APOB | Wild-type reporter | S | APOB WT Sense | tcgagCTTGAATTCCAAGAGCACACGGTCTTCAGTGAAGCTGCAGGg | 150 |
| | | AS | APOB WT Anti-sense | aattcCCTGCAGCTTCACTGAAGACCGTGTGCTCTTGGAATTCAAGc | 151 |
| | Mutant reporter | S | APOB 10580 Sense | tcgagCTTGAATTCCAAGAGCACACAGTCTTCAGTGAAGCTGCAGGg | 152 |
| | | AS | APOB 10580 Anti-sense | aattcCCTGCAGCTTCACTGAAGACTGTGTGCTCTTGGAATTCAAGc | 153 |
| F12 (CG) | Wild-type reporter | S | F12 WT Sense | tcgagACCGCCGAAGCCTCAGCCCACGACCCGGACCCCGCCTCAGTg | 154 |
| | | AS | F12 WT Anti-sense | aattcACTGAGGCGGGGTCCGGGTCGTGGGCTGAGGCTTCGGCGGTc | 155 |
| | Mutant reporter | S | F12 983 CG Sense | tcgagACCGCCGAAGCCTCAGCCCAGGACCCGGACCCCGCCTCAGTg | 156 |
| | | AS | F12 983 CG Anti-sense | aattcACTGAGGCGGGGTCCGGGTCCTGGGCTGAGGCTTCGGCGGTc | 157 |
| CLCN7 | Wild-type reporter | S | CLCN7 WT Sense | tcgagTGTTCCGGGCCCTGGGCCTGCGGCACCTGGTGGTGGTGGACg | 158 |
| | | AS | CLCN7 WT Anti-sense | aattcGTCCACCACCACCAGGTGCCGCAGGCCCAGGGCCCGGAACAc | 159 |
| | Mutant reporter | S | CLCN7 2299 Sense | tcgagTGTTCCGGGCCCTGGGCCTGTGGCACCTGGTGGTGGTGGACg | 160 |
| | | AS | CLCN7 2299 Anti-sense | aattcGTCCACCACCACCAGGTGCCACAGGCCCAGGGCCCGGAACAc | 161 |
| SCN8A | Wild-type reporter | S | SCN8A WT Sense | tcgagTCCCCTCCATCATGAATGTGCTGCTGGTGTGTCTCATCTTCg | 162 |
| | | AS | SCN8A WT Anti-sense | aattcGAAGATGAGACACACCAGCAGCACATTCATGATGGAGGGGAc | 163 |
| | Mutant reporter | S | SCN8A 3991 Sense | tcgagTCCCCTCCATCATGAATGTGGTGCTGGTGTGTCTCATCTTCg | 164 |
| | | AS | SCN8A 3991 Anti-sense | aattcGAAGATGAGACACACCAGCACCACATTCATGATGGAGGGGAc | 165 |
| PCSK9 | Wild-type reporter | S | PCSK9 WT Sense | tcaaaACATCATTGGTGCCTCCAGCGACTGCAGCACCTGCTTTGTGa | 166 |
| | | AS | PCSK9 WT Anti-sense | aattcCACAAAGCAGGTGCTGCAGTCGCTGGAGGCACCAATGATGTc | 167 |
| | Mutant reporter | S | PCSK9 1120 Sense | tcaaaACATCATTGGTGCCTCCAGCTACTGCAGCACCTGCTTTGTGa | 168 |
| | | AS | PCSK9 1120 Anti-sense | aattcCACAAAGCAGGTGCTGCAGTAGCTGGAGGCACCAATGATGTc | 169 |
| KRT6A | Wild-type reporter | S | KRT6A WT Sense | tcaaaTCAAGACCCTCAACAACAAGTTTGCCTCCTTCATCGACAAGa | 170 |
| | | AS | KRT6A WT Anti-sense | aattcCTTGTCGATGAAGGAGGCAAACTTGTTGTTGAGGGTCTTGAc | 171 |
| | Mutant reporter | S | KRT6A 520 Sense | tcqaqTCAAGACCCTCAACAACAAGGTTGCCTCCTTCATCGACAAGg | 172 |
| | | AS | KRT6A 520 Anti-sense | aattcCTTGTCGATGAAGGAGGCAACCTTGTTGTTGAGGGTCTTGAc | 173 |

For siRNAs, the base at position 11 of each gene corresponded to the position of the point mutation in the corresponding mutant allele, the group at the 2'-position of the pentose in each of the ribonucleotides at positions 6-8 of the siRNA's guide strand was replaced by OCH₃, and the base at position 6 of the guide strand was mismatched with that of the corresponding mutant allele. The 5'-ends of the guide and passenger strands of each siRNA were replaced as indicated in Table 3.

**[Table 3]**

| Gene Name (Position of mutation) | Base change at 5'-end of G strand | Base change at 5'-end of P strand |
|---|---|---|
| HTT (rs363125) | - | C-G |
| HTT (rs362307) | G-U | - |
| HTT (rs362331) | - | C-G |
| ATXN3 | G-U | A-G |
| ZMYM3 | A→U | U-G |
| CTNNB1 | G-U | - |
| SMARCA4 | C-U | - |
| SMO | C-G | - |
| AR | G-U | U-G |
| DNM2 | G-U | U-G |
| KRT14 | G-U | - |
| IL4R (223) | - | A-G |
| IL4R (1507) | - | U-G |
| MAPT | G-U | U-G |
| MS4A2 | A→U | C-G |
| PABPN1 | G-U | A-G |
| RHO(68) | G-U | A-G |
| RHO (1039) | - | A-G |
| SCNIA | - | - |
| APOB | C-U | - |
| F12 | G-U | U-G |
| CLCN7 | A→U | U-G |
| SCN8A | C-U | U-G |
| PCSK9 | G-U | C-G |
| KRT6A | A→U | A-G |

The reporters indicated in Table 2 were used for the corresponding genes, and reporter assays were performed using siRNAs indicated in Tables 4 and 5 using the aforementioned method.

**[Table 4]**

| Gene Name (Position of mutation) | Name | Strand | Sequence | Seq. ID No. |
|---|---|---|---|---|
| HTT (rs363125) | siHTT_363125-WT | P | CCACAGUACUUCAACGCUAGA | 174 |
| | | G | UAGCGUUGAAGUACUGUGGGG | 175 |
| HTT (rs362307) | siHTT_362307-WT | P | GAAGUCUGCGCCCUUGUGCCC | 176 |
| | | G | GCACAAGGGCGCAGACUUCCA | 177 |
| HTT (rs362331) | siHTT_362331-WT | P | CCCUCAUCUACUGUGUGCACU | 178 |
| | | G | UGCACACAGUAGAUGAGGGAG | 179 |
| ATXN3 | siATXN3-WT | P | AGCAGCAGGGGGACCUAUCAG | 180 |
| | | G | GAUAGGUCCCCCUGCUGCUGC | 181 |
| ZMYM3 | siZMYM3_WT | P | UCCACUGGCGUGGGCAGAUCC | 182 |
| | | G | AUCUGCCCACGCCAGUGGAUG | 183 |
| CTNNB1 | siCTNNB1_WT | P | GUGCCACUACCACAGCUCCUU | 184 |
| | | G | GGAGCUGUGGUAGUGGCACCA | 185 |
| SMARCA4 | siSMARCA4_WT | P | GCUGCUGACGGGCACACCGCU | 186 |
| | | G | CGGUGUGCCCGUCAGCAGCAG | 187 |
| SMO | siSMO_WT | P | GCCUGGUGCUCAUCGUGGGAG | 188 |
| | | G | CCCACGAUGAGCACCAGGCCG | 189 |

**[Table 5]**

| Gene Name (Position of mutation) | Name | Strand | Sequence | Seq. ID No. |
|---|---|---|---|---|
| HTT (rs363125) | siHTT_363125-SNP | P | GCACAGUAAUUCAUCGCUAGA | 190 |
| | | G | UAGCGAUGAAUUACUGUGCGG | 191 |
| HTT (rs362307) | siHTT_362307-SNP | P | GAAGUCUGUGCCCAUGUGACC | 192 |
| | | G | UCACAUGGGCACAGACUUCCA | 193 |
| HTT (rs362331) | siHTT_362331-SNP | P | GCCUCAUCCACUGAGUGCACU | 194 |
| | | G | UGCACUCAGUGGAUGAGGCAG | 195 |
| ATXN3 | siATXN3-SNP | P | GGCAGCAGCGGGAGCUAUAAG | 196 |
| | | G | UAUAGCUCCCGCUGCUGCCGC | 197 |
| ZMYM3 | siZMYM3_MUT_2206 | P | GCCACUGGUGUGGCCAGAACC | 198 |
| | | G | UUCUGGCCACACCAGUGGCUG | 199 |
| CTNNB1 | siCTNNB1_MUT_121 | P | GUGCCACUGCCACUGCUCAUU | 200 |
| | | G | UGAGCAGUGGCAGUGGCACCA | 201 |
| SMARCA4 | siSMARCA4_MUT_2729 | P | GCUGCUGAUGGGCUCACCACU | 202 |
| | | G | UGGUGAGCCCAUCAGCAGCAG | 203 |
| SMO | siSMO_MUT_1234 | P | GCCUGGUGUUCAUGGUGGAAG | 204 |
| | | G | GCCACCAUGAACACCAGGCCG | 205 |
| AR | siAR_MUT_2180 | P | GGGCUUCCUCAACAUACAAGU | 206 |
| | | G | UUGUAUGUUGAGGAAGCCCGG | 207 |
| DNM2 | siDNM2_MUT_1102 | P | GCUUCCACAAGCGCUUCCAAU | 208 |
| | | G | UGGAAGCGCUUGUGGAAGCUG | 209 |
| KRT14 | siKRT14_MUT_1151 | P | GGAGCAGCCGGCCGAGCUACG | 210 |
| | | G | UAGCUCGGCCGGCUGCUCCUC | 211 |
| IL4R (223) | silLR4_MUT_223 | P | GCACGUGUGUCCCAGAGAACA | 212 |
| | | G | UUCUCUGGGACACACGUGCGG | 213 |
| IL4R (1507) | silLR4_MUT_1507 | P | GCAGCAACCCCCUCAGCCAGU | 214 |
| | | G | UGGCUGAGGGGGUUGCUGCAG | 215 |
| MAPT (1853) | siMAPT_MUT_1853 | P | GCACGUCCUGGGACGCGGAAG | 216 |
| | | G | UCCGCGUCCCAGGACGUGCUU | 217 |
| MS4A2 | siMS4A2_MUT_710 | P | GCCAGGGGGAAUGACUCCACC | 218 |
| | | G | UGGAGUCAUUCCCCCUGGCUC | 219 |
| PABPN1 | siPABPN1_MUT_35 | P | GGCAGCGGCGGCUCCGGGAGG | 220 |
| | | G | UCCCGGAGCCGCCGCUGCCGC | 221 |
| RHO (68) | siRHO_MUT_68 | P | GCGCAGCCACUUCCAGUAACC | 222 |
| | | G | UUACUGGAAGUGGCUGCGCAC | 223 |
| RHO (1039) | siRHO_MUT_1039 | P | GGGUGGCCUCGGCGUAAGACC | 224 |
| | | G | UCUUACGCCGAGGCCACCCGG | 225 |
| SCNIA | siSCN1A_MUT_664 | P | GAGUUCUCUGAGCUUUGAAGA | 226 |
| | | G | UUCAAAGCUCAGAGAACUCUG | 227 |
| APOB | siAPOB_MUT_10580 | P | GAGCACACAGUCUACAGUAAA | 228 |
| | | G | UACUGUAGACUGUGUGCUCUU | 229 |
| F12 (983 CG) | siF12_MUT_983 CG | P | GCAGCCCAGGACCGGGACACC | 230 |
| | | G | UGUCCCGGUCCUGGGCUGCGG | 231 |
| CLCN7 | siCLCN7_MUT_2299 | P | GGACCCGGACGCCGUGGACCA | 323 |
| | | G | UCCAGCUGCCACAGGCCCCGG | 233 |
| SCN8A | siSCN8A_MUT_3991 | P | GGAAUGUGGUGCUCGUGUAUC | 234 |
| | | G | UACACGAGCACCACAUUCCUG | 235 |
| PCSK9 | siPCSK9_MUT_1120 | P | GCUCCAGCUACUGGAGCAACU | 236 |
| | | G | UUGCUCCAGUAGCUGGAGCCA | 237 |
| KRT6A | siKRT6A_MUT_520 | P | GCAACAAGGUUGCGUCCUACA | 238 |
| | | G | UAGGACGCAACCUUGUUGCUG | 239 |

Figs. 11 to 35 show the results of the assays. For all genes, the siRNAs suppressed the expressions of the mutant alleles in a concentration-dependent manner, though the expressions of the wild-type alleles were hardly suppressed.

### Industrial applicability

The present invention allowed to provide novel RNA molecules, novel chimeric NA molecules, novel double-stranded RNA molecules, and novel double-stranded chimeric NA molecules.

## Claims

1. An RNA molecule for use in RNA interference to target a mutant allele of a gene, the mutant allele having a point mutation relative to a wild-type allele of the gene, wherein the gene is selected from the group consisting of *HTT*, *ATXN3*, *ZMYM3, CTNNB1*, *SMARCA4, SMO, AR*, *DNM2, KRT14*, *IL4R, MAPT*, *MS4A2*, *PABPN1, RHO, SCNIA, APOB, F12, CLCN7, SCN8A, PCSK9,* and *KRT6A,* and wherein the RNA molecule satisfies the followings:
(1) the molecule has a nucleotide sequence complementary to a nucleotide sequence of a coding region of the mutant allele except for a base specified in (2-1) below; and
(2) when counted from the base at the 5'-end of a nucleotide sequence complementary to the nucleotide sequence of the mutant allele,
(2-1) a base at position 5 or 6 is mismatched with a base in the mutant allele;
(2-2) a base at position 10 or 11 is at the position of the point mutation and is identical to the base at the position of the point mutation in the mutant allele; and
(2-3) the group at the 2'-position of the pentose in each of ribonucleotides at positions 6-8 or positions 7 and 8 is independently modified with OCH₃, halogen, or LNA.

2. The RNA molecule according to Claim 1, wherein the halogen is fluorine.

3. The RNA molecule according to Claim 1 or 2, wherein, when the base at the 5'-end of the nucleotide sequence specified in (1) of Claim 1 is cytosine or guanine, the base is replaced by adenine or uracil.

4. The RNA molecule according to any one of Claims 1 to 3, wherein, when the base at the 3'-end of the nucleotide sequence specified in (1) of Claim 1 is adenine or uracil, it is replaced by cytosine or guanine.

5. The RNA molecule according to any one of Claims 1 to 4, wherein the RNA molecule comprises 13-28 nucleotides.

6. The RNA molecule according to any one of Claims 1 to 5, further comprising 1-3 nucleotide(s) at the 3'-end of the nucleotide sequence specified in (1) of Claim 1.

7. A chimeric NA molecule, wherein one or more ribonucleotides in an RNA molecule according to any one of Claims 1 to 6 are each replaced by a deoxyribonucleotide, an artificial nucleic acid, or a nucleic acid analog.

8. A double-stranded RNA molecule comprising a guide strand and a passenger strand, wherein the guide strand is an RNA molecule according to any one of Claims 1 to 5, and the passenger strand is an RNA molecule with a sequence complementary to the sequence of the RNA molecule of the guide strand.

9. The double-stranded RNA molecule according to Claim 8, wherein the RNA molecule comprises an overhang at the 3'-end of the guide strand and/or an overhang at the 3'-end of the passenger strand.

10. The double-stranded RNA molecule according to Claim 9, wherein either or both of the overhangs comprise 1-3 nucleotides.

11. A double-stranded chimeric NA molecule, wherein one or more ribonucleotides in a double-stranded RNA molecule according to any one of Claims 8 to 10 are each replaced by a deoxyribonucleotide, an artificial nucleic acid, or a nucleic acid analog.

12. A method for producing an RNA molecule for use as a guide strand in RNA interference, comprising the step of:
producing an RNA molecule according to any one of Claims 1 to 6.

13. A method for producing a chimeric NA molecule for use as a guide strand in RNA interference, comprising the step of:
producing a chimeric NA molecule according to Claim 7.

14. A method for performing RNA interference to target a mutant allele of a gene in a cell containing a wild-type allele of the gene and the mutant allele, the mutant allele having a point mutation, the method comprising the step of:
introducing an RNA molecule according to any one of Claims 1 to 6, a chimeric NA molecule according to Claim 7, a double-stranded RNA molecule according to any one of Claims 8 to 10, or a double-stranded chimeric NA molecule according to Claim 11 into the cell.

15. A therapeutic agent for a patient with a disease or a prophylactic agent for a carrier of the disease, the patient or the carrier having wild-type and mutant alleles of a causative gene for the disease, the mutant allele having a point mutation and being responsible for the disease, wherein:
the therapeutic agent or the prophylactic agent comprises, as an active ingredient, an RNA molecule according to any one of Claims 1 to 6, a chimeric NA molecule according to Claim 7, a double-stranded RNA molecule according to any one of Claims 8 to 10, or a double-stranded chimeric NA molecule according to Claim 11.

16. The therapeutic agent according to Claim 15, wherein the disease is a trinucleotide repeat disease, a genetic disease or a tumor.

17. A method for selecting an RNA molecule, a chimeric NA molecule, a double-stranded RNA molecule, or a double-stranded chimeric NA molecule for use in RNA interference to silence a target gene, the method comprising the steps of:
evaluating a gene-specific silencing ability of a plurality of the RNA molecules, the chimeric NA molecules, the double-stranded RNA molecules, or the double-stranded chimeric NA molecules, to the target gene by performing the method according to Claim 11 *in vitro* using each of the plurality of the RNA molecules according to any one of Claims 1 to 6, the chimeric NA molecules according to Claim 7, the double-stranded RNA molecules according to any one of Claims 8 to 10, or the double-stranded chimeric NA molecules according to Claim 11; and
selecting an RNA molecule, a chimeric NA molecule, a double-stranded RNA molecule, or a double-stranded chimeric NA molecule having at least a certain level of the gene-specific silencing ability.
